(19) Europäisches Patentamt / European Patent Office / Office européen des brevets

(11) EP 1 737 945 B1

(12) EUROPEAN PATENT SPECIFICATION

(45) Date of publication and mention of the grant of the patent:
**26.01.2011 Bulletin 2011/04**

(21) Application number: **05739527.9**

(22) Date of filing: **19.04.2005**

(51) Int Cl.:
*C12N 1/06* (2006.01) *C12N 15/10* (2006.01)
*C12Q 1/68* (2006.01)

(86) International application number:
**PCT/EP2005/005213**

(87) International publication number:
**WO 2005/100542 (27.10.2005 Gazette 2005/43)**

(54) **METHOD FOR PURIFYING PLASMID DNA**

VERFAHREN ZUR HERSTELLUNG VON PLASMID DNS IN PHARMAZEUTISCHER QUALITÄT

PROCEDE DESTINE A PURIFIER DE L'ADN PLASMIDIQUE

(84) Designated Contracting States:
**AT BE BG CH CY CZ DE DK EE ES FI FR GB GR HU IE IS IT LI LT LU MC NL PL PT RO SE SI SK TR**

(30) Priority: **19.04.2004 US 563008 P**
**17.09.2004 PCT/EP2004/011437**

(43) Date of publication of application:
**03.01.2007 Bulletin 2007/01**

(60) Divisional application:
**10157884.7 / 2 246 413**

(73) Proprietor: **Aventis Pharma S.A.**
**92160 Antony (FR)**

(72) Inventors:
- **BLANCHE, Francis**
**F-75019 Paris (FR)**
- **COUDER, Michel**
**F-94370 Sucy-en-Brie (FR)**
- **MAESTRALI, Nicolas**
**F-91650 Breuillet (FR)**
- **GUILLEMIN, Thierry**
**F-91590 Boissy Le Cutte (FR)**
- **GAILLAC, David**
**F-69008 Lyon (FR)**

(74) Representative: **Bouvet, Philippe**
**Sanofi Aventis**
**Patents France**
**174 avenue de France**
**75013 Paris (FR)**

(56) References cited:
**WO-A-97/23601 WO-A-2005/026331**

- **FERREIRA G N M ET AL: "DOWNSTREAM PROCESSING OF PLASMID DNA FOR GENE THERAPY AND DNA VACCINE APPLICATIONS" TRENDS IN BIOTECHNOLOGY, ELSEVIER PUBLICATIONS, CAMBRIDGE, GB, vol. 18, September 2000 (2000-09), pages 380-388, XP000972180 ISSN: 0167-7799**
- **COMEROTA A J ET AL: "Naked plasmid DNA encoding fibroblast growth factor type 1 for the treatment of end-stage unreconstructible lower extremity ischemia: Preliminary results of a phase I trial" BIOSIS, May 2002 (2002-05), XP002300322**
- **STADLER J ET AL: "Plasmid DNA purification" JOURNAL OF GENE MEDICINE, WILEY,, US, vol. 6, no. SUPPL 1, February 2004 (2004-02), pages S54-S66, XP002272777 ISSN: 1099-498X**
- **STEMMER W P C ET AL: "Single-step assembly of a gene and entire plasmid from large numbers of oligodeoxyribonucleotides" GENE, ELSEVIER BIOMEDICAL PRESS. AMSTERDAM, NL, vol. 164, no. 1, 1995, pages 49-53, XP004041916 ISSN: 0378-1119**
- **PRAZERES D.M.F. AND FERREIRA G.N.M.: "Design of flowsheets for the recovery and purification of plasmids for gene therapy and DNA vaccination" CHEMICAL ENGINEERING AND PROCESSING, vol. 43, 8 October 2003 (2003-10-08), pages 615-630, XP002334410**
- **COSTIOLI MATTEO D ET AL: "DNA purification by triple-helix affinity precipitation." BIOTECHNOLOGY AND BIOENGINEERING. 5 MAR 2003, vol. 81, no. 5, 5 March 2003 (2003-03-05), pages 535-545, XP002334411 ISSN: 0006-3592**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- SCHLUEP T ET AL: "purification of plasmids by triplex affinity interaction" NUCLEIC ACIDS RESEARCH, OXFORD UNIVERSITY PRESS, SURREY, GB, vol. 26, no. 19, 1998, pages 4524-4528, XP002219768 ISSN: 0305-1048
- WILS P ET AL: "EFFICIENT PURIFICAITON OF PLASMID DNA FOR GENE TRANSFER USING TRIPLE-HELIX AFFINITY CHROMATOGRAPHY" GENE THERAPY, MACMILLAN PRESS LTD., BASINGSTOKE, GB, vol. 4, no. 4, 1 April 1997 (1997-04-01), pages 323-330, XP000675208 ISSN: 0969-7128

SEQUENCE LISTING

<110> CENTELION

<120> Method for Purifying Plasmid DNA

<130> GC03001A

<150> PCT/EP2004/011437
<151> 2004-09-17

<150> US 60/563,008
<151> 2004-04-19

<160> 34

<170> PatentIn version 3.2

<210> 1
<211> 25
<212> DNA
<213> Artificial

<220>
<223> Synthetic Oligonucleotide

<400> 1
gaggcttctt cttcttcttc ttctt 25

<210> 2
<211> 19
<212> DNA
<213> Artificial

<220>
<223> Synthetic oligonucleotide

<400> 2
aagggaggga ggagaggaa 19

<210> 3
<211> 19
<212> DNA
<213> Artificial

<220>
<223> Synthetic oligonucleotide

<400> 3
aaggagagga gggagggaa 19

<210> 4
<211> 19
<212> DNA
<213> Artificial

<220>
<223> Synthetic oligonucleotide

<400> 4
ttggtgtggt gggtgggtt 19

<210> 5
<211> 19
<212> DNA
<213> Artificial

<220>
<223> Synthetic oligonucleotide

<400> 5
cttcccgaag ggagaaagg 19

<210> 6
<211> 19
<212> DNA
<213> Artificial

<220>
<223> Synthetic oligonucleotide

<400> 6
gaagggcttc cctctttcc 19

<210> 7
<211> 13
<212> DNA
<213> Artificial

<220>
<223> Synthetic oligonucleotide

<400> 7
gaaaaaggaa gag 13

<210> 8
<211> 12
<212> DNA
<213> Artificial

<220>
<223> Synthetic oligonucleotide

<400> 8
agaaaaaaag ga 12

<210> 9
<211> 12
<212> DNA
<213> Artificial

<220>
<223> Synthetic oligonucleotide

<400> 9
tcttttttc ct 12

<210> 10
<211> 14
<212> DNA

<213> Artificial

<220>
<223> Synthetic oligonucleotide

<400> 10
aagaaaaaaa agaa 14

<210> 11
<211> 14
<212> DNA
<213> Artificial

<220>
<223> Synthetic nucleotide

<400> 11
ttcttttttt tctt 14

<210> 12
<211> 17
<212> DNA
<213> Artificial

<220>
<223> Synthetic oligonucleotide

<400> 12
aaaaaaggga ataaggg 17

<210> 13 <211> 58
<212> DNA
<213> Artificial

<220>
<223> Synthetic oligonucleotide

<400> 13
gatccgaaga agaagaagaa gaagaagaag aagaagaaga agaagaagaa gaagaagg 58

<210> 14
<211> 58
<212> DNA
<213> Artificial

<220>
<223> Synthetic oligonucleotide

<400> 14
aattccttct tcttcttctt cttcttcttc ttcttcttct tcttcttctt cttcttcg 58

<210> 15
<211> 51
<212> DNA
<213> Artificial

<220>
<223> Synthetic oligonucleotide

<400> 15
gaagaagaag aagaagaaga agaagaagaa gaagaagaag aagaagaaga a 51

<210> 16
<211> 17
<212> DNA
<213> Artificial

<220>
<223> Synthetic oligonucleotide

<400> 16
tgaccggcag caaaatg 17

<210> 17
<211> 27
<212> DNA
<213> Artificial

<220>
<223> Synthetic oligonucleotide

<400> 17
ccgaattctg gggaccaaag cagtttc 27

<210> 18
<211> 27
<212> DNA
<213> Artificial

<220>
<223> Synthetic oligonucleotide

<400> 18
ccaagcttca ctgttcacga cgggtgt 27

<210> 19
<211> 25
<212> DNA
<213> Artificial

<220>
<223> 5-methylcytosine

<400> 19
gaggcttctt cttcttcttc ttctt 25

<210> 20
<211> 48
<212> DNA
<213> Artificial

<220>
<223> Synthetic oligonucleotide

<400> 20
ggaggaggag gaggaggagg aggaggagga ggaggaggag gaggagga 48

<210> 21
<211> 50
<212> DNA
<213> Artificial

<220>
<223> Synthetic oligonucleotide

<400> 21
gagagagaga gagagagaga gagagagaga gagagagaga gagagagaga 50

<210> 22
<211> 58
<212> DNA
<213> Artificial

<220>
<223> Synthetic oligonucleotide

<400> 22
gatccgagag agagagagag agagagagag agagagagag agagagagag agagaggg 58

<210> 23
<211> 58
<212> DNA
<213> Artificial

<220>
<223> Synthetic oligonucleotide

<400> 23
aattccctct ctctctctct ctctctctct ctctctctct ctctctctct ctctctcg 58

<210> 24
<211> 58
<212> DNA
<213> Artificial

<220>
<223> Synthetic oligonucleotide

<400> 24
gatccggagg aggaggagga ggaggaggag gaggaggagg aggaggagga ggaggagg 58

<210> 25
<211> 58
<212> DNA
<213> Artificial

<220>
<223> Synthetic oligonucleotide

<400> 25
aattcctcct cctcctcctc ctcctcctcc tcctcctcct cctcctcctc ctcctccg 58

<210> 26
<211> 50
<212> DNA

<213> Artificial

<220>
<223> Synthetic oligonucleotide

<400> 26
ggagaggagg aggaggagga ggaggaggag gaggaggagg aggaggagga 50

<210> 27
<211> 25
<212> DNA
<213> Artificial

<220>
<223> Synthetic oligonucleotide

<400> 27
aatgcctcct cctcctcctc ctcct 25

<210> 28
<211> 26
<212> DNA
<213> Artificial

<220>
<223> Synthetic oligonucleotide

<400> 28
agtgctctct ctctctctct ctctct 26

<210> 29
<211> 66
<212> DNA
<213> Artificial

<220>
<223> Synthetic oligonucleotide

<400> 29

gatctgaaga agaagaagaa gaagaagaag aagaagaaga agaagaagaa gaagaactgc 60
agatct 66

<210> 30
<211> 66
<212> DNA
<213> Artificial

<220>
<223> Synthetic oligonucleotide

<400> 30

gatcagatct gcagttcttc ttcttcttct tcttcttctt cttcttcttc ttcttcttct 60

tcttca 66

<210> 31
<211> 21
<212> DNA
<213> Artificial

<220>
<223> Synthetic oligonucleotide

<400> 31
acagtcataa gtgcggcgac g 21

<210> 32
<211> 39
<212> DNA
<213> Artificial

<220>
<223> Synthetic oligonucleotide

<400> 32
gaagaagagg aagaagaaga agaagaagaa ggaagagaa 39

<210> 33
<211> 21
<212> DNA
<213> Artificial

<220>
<223> Synthetic oligonucleotide

<400> 33
gaagaagaag aagaagaaga a 21

<210> 34
<211> 12
<212> DNA
<213> Artificial

<220>
<223> Synethtic oligonucleotide

<400> 34
agaaaaaaaa ga 12

## Description

### FIELD OF THE INVENTION

This invention relates to methods for purifying nucleic acids. The invention relates in particular to methods for preparing highly purified plasmid DNA (pDNA), in particular to the production and isolation of pharmaceutical grade plasmid DNA.

### BACKGROUND OF THE INVENTION

Developments in molecular biology clearly suggest that plasmid-based therapy in particular in the field vaccines and human gene therapy may support effective ways to treat diseases. A significant hurdle to this technology, however, is the preparation of plasmid DNA sufficient in quantity and quality for clinical use. One promising method of safely and effectively delivering a normal gene into human cells is via plasmid DNA. Plasmid DNA is a closed, circular form of bacterial DNA into which a DNA sequence of interest can be inserted. Examples of DNA sequences of interest that may be introduced in mammalian cells include exogenous, functional gene, or mutant gene, antisense sequences, RNAi or dsRNAi sequences, ribozymes, for example in the treatment of viral infections, cancer or angiogenesis- related diseases. Once delivered to the human cell, the pDNA begins replicating and producing copies of the inserted DNA sequence. Thus, scientists view plasmid DNA as a promising vehicle for delivery of DNA sequences of interest into human cells in order to treat a variety of disease states.

Huge quantities of plasmid DNA are needed for research development to implement plasmid-based technology in a therapeutic context. Since the plasmid DNA used in gene therapy and other clinical applications is usually produced by bacteria such as *Escherichia coli* (*E. coli*), methods are needed to effectively separate the plasmid DNA from the genomic DNA (gDNA) of the bacterial cell, as well as from endotoxin and proteins in the bacterial cell. Thus, there is a growing need for simple, robust, and scalable purification processes that can be used to isolate large amounts of plasmid DNA from bacterial cells.

An important step in any plasmid purification process involves the lysis of bacterial cells in order to release the cellular contents from which the pDNA can then be isolated. In fact, it is first necessary to achieve three steps of cell resuspension, cells lysis and neutralization and precipitation of host contaminants. Cell resuspension normally utilizes manual stirring or a magnetic stirrer, and a homogenizer or impeller mixer to resuspend cells in the resuspension buffer. Cell lysis may carried out by manual swirling or magnetic stirring in order to mix the resuspended cells with lysis solution (consisting of diluted alkali (base) and detergents); then holding the mixture at room temperature (20-25 degrees Celsius) or on ice for a period of time, such as 5 minutes, to complete lysis. As noted above, manual swirling and magnetic stirring are not scalable. The third stage is neutralization and precipitation of host contaminants. Lysate from the second stage is normally mixed with a cold neutralization solution by gentle swirling or magnetic stirring to acidify the lysate before setting in ice for 10-30 minutes to facilitate the denaturation and precipitation of high molecular weight chromosomal DNA, host proteins, and other host molecules. Both manual swirling and magnetic stirring are not scalable.

Generally, the cell wall is digested by treating with lysozyme for a short time or via alkaline or potassium acetate (KOAc) treatment. RNase is also generally added to degrade RNAs of the bacterial suspension. These chemical steps may be efficient in lysing cells on a small scale. However, the increase in viscosity makes large scale processing very difficult.

An alternative simple and rapid method for preparing plasmids comprises lysozyme treatment of the bacteria, then boiled at about 100°C in an appropriate buffer for 20 to 40 seconds forming an insoluble clot of genomic DNA, protein and debris leaving the plasmid in solution with RNA as the main contaminant. Next, a mixed solution of NaOH and sodium dodecylsulfate (SDS) is added for the purpose of dissolving the cytoplasmic membrane. NaOH partially denatures DNAs and partially degrades RNAs and SDS acts to dissolve the membrane and denature proteins. Successively, SDS-protein complex and cell debris are precipitated by adding 5N potassium acetate (pH 4.8). At this time, pH is important for both to neutralize NaOH used in said manipulation and to renature plasmid. Thereafter, centrifugation is applied to remove the precipitates, thus obtaining aiming plasmids in supernatant. However, this technique is not suitable for scale up to a high volume of bacterial fermentations and is meant for fermentations of less than five liters. Also, these series of manipulations require to mix slowly and firmly, so as to avoid that the bacterial chromosomal DNA is cut off to small fragments and aggregate, causing them to contaminate the plasmid, and difficult to implement on a large scale processing.

One common alternative method of lysing cells, known as alkaline lysis, consists of mixing a suspension of bacterial cells (solution 1) with an alkaline lysis solution (solution 2). Solution 2 consists of a detergent, e.g., sodium dodecyl sulfate (SDS), to lyse the bacterial cells and release the intracellular material, and an alkali, e.g., sodium hydroxide, to denature the proteins and nucleic acids of the cells (particularly gDNA and RNA). As the cells are lysed and the DNA is denatured, the viscosity of the solution rises dramatically. After denaturation, an acidic solution, e.g.,

potassium acetate (solution 3), is added to neutralize the sodium hydroxide, inducing renaturation of nucleic acids. The long fragments of gDNA reassociate randomly and form networks that precipitate as flocs, entrapping proteins, lipids, and other nucleic acids. The potassium salt of dodecyl sulfate also precipitates, carrying away the proteins with which it is associated. The two strands of pDNA (plasmid DNA), intertwined with each other, reassociate normally to reform the initial plasmid, which remains in solution.

This lysis technique is conducted in batch mode, *i.e.,* where the different solutions are mixed by sequentially adding the solutions to vessels or tanks. Because the alkaline lysate is a viscoelastic fluid that is very difficult to manipulate, one difficulty with this method occurs during the mixing of the different solutions. Since shear stress causes fragmentation of gDNA, which then becomes extremely difficult to separate from pDNA, methods are needed to avoid application of shear stresses to the fluid. In addition, large pDNA (i.e. greater than about 10 kilo base pairs) is also susceptible to shear damage during the mixing process. After the solution containing the cell suspension has been mixed with the lysis solution, the viscoelastic alkaline lysate is mixed with the neutralization solution. Again, this mixing process is problematic due to the viscoelastic properties of the solution.

In addition, another difficulty in scaling up the batch lysis process involves the efficiency of mixing of the different fluids while attempting to limit the shear stresses so as to avoid fragmenting gDNA. As noted previously, the chromatographic behavior of fragmented genomic DNA is very similar to that of pDNA, so that it becomes virtually impossible to get rid of gDNA by standard purification procedures. Thus, several limitations of using a batch process to lyse bacterial cells are apparent, such as scaling up, poor quality of the recovered pDNA due to contamination by fragmented gDNA, and the relatively low quantity of pDNA obtained.

In contrast to the batch method, several methods for continuously mixing various cell-lysis solutions using a series of static mixers have also been proposed. According to these methods, a cell suspension solution and a cell-lysing solution are simultaneously added to a static mixer. The lysed cell solution that exits the first static mixer and a precipitating solution are then simultaneously added to a second static mixer. The solution that exits this second mixer contains the precipitated lysate and plasmids. Other continuous modes of lysing cells include use of a flow-through heat exchanger where the suspended cells are heated to 70-100°C. Following cell lysis in the heat exchanger, the exit stream is subjected to either continuous flow or batch-wise centrifugation during which the cellular debris and genomic DNA are precipitated, leaving the plasmid DNA in the supernatant.

Large scale isolation and purification of plasmid DNA from large volume microbial fermentations therefore requires the development of an improved plasmid preparation process.

Despite the numerous methods currently used to lyse bacterial cells, none of them address the problems caused by the viscoelastic properties of the fluids and the shear forces involved during mixing steps. The present invention thus relates to a novel method for continuous alkaline lysis of the bacterial cell suspension at a large scale and provides with a major advantage in limiting shear forces.

Another important step for any application in which nucleic acid is introduced into a human or animal in a therapeutic context is the need to produce highly purified, pharmaceutical grade nucleic acid. Such purified nucleic acid must meet drug quality standards of safety, potency and efficacy. In addition, it is desirable to have a scaleable process that can be used to produce multiple gram quantities of DNA. Thus, it is desirable to have a process for producing highly pure nucleic acid that does not require toxic chemicals, mutagens, organic solvents, or other reagents that would compromise the safety or efficacy of the resulting nucleic acid, or make scale-up difficult or impractical. It is also desirable to prepare nucleic acids free from contaminating endotoxins, which if administered to a patient could elicit a toxic response. Removal of contaminating endotoxins is particularly important where plasmid DNA is purified from gram-negative bacterial sources that have high levels of endotoxins as an integral component of the outer cell membrane'.

The classical techniques for isolating and purifying plasmid DNA from bacterial fermentations are suitable for small or laboratory scale plasmid preparations. After disruption of bacterial host cells containing the plasmid, followed by acetate neutralization causing the precipitation of host cell genomic DNA and proteins are generally removed by, for example, centrifugation. The liquid phase contains the plasmid DNA which is alcohol precipitated and then subjected to isopycnic centrifugation using CsCl in the presence of ethidium bromide to separate the various forms of plasmid DNA, *i.e.*, supercoiled, nicked circle, and linearized. Further extraction with butanol is required to remove residual ethidium bromide followed by DNA precipitation using alcohol. Additional purification steps follow to remove host cell proteins.

These current methods for isolating plasmid DNA have several limitations. For example, purification methods that involve the use of large amounts of flammable organic solvents (e.g., ethanol and isopropanol) and toxic chemicals, *e.g.*, ethidium bromide, phenol, and chloroform, are generally undesirable for large scale isolation and purification of plasmid DNA. Alternatives methods to the cesium chloride centrifugation may be used for plasmid DNA purification, such as size exclusion chromatography, chromatography on hydroxyapatite, and various chromatographic methods based on reverse phase or anion exchange. These alternatives may be adequate to produce small amounts of research material on a laboratory scale, but are generally not easily scaleable and are not capable of producing the quantities of plasmid DNA.

Also, with the chemical separating method, separating and purifying process is complicated and a large quantity

of organic solvent must be used, hence it poses many problems of treatment of waste solvents and others.

Besides the chemical separating and purifying method, there is a method of separating plasmids by electrophoresis. The electrophoretic method includes paper electrophoresis and gel electrophoresis, and gel electrophoresis is common currently. However, the electrophoretic method has many problems of long separation time, difficult collection, low sample loading, etc.

Currently available methods for separation of the two forms of plasmid DNA utilize ion exchange chromatography (Duarte et al., Journal of Chromatography A, 606 (1998), 31-45) or size exclusion chromatography (Prazeres, D.M., Biotechnology Techniques Vol. 1, No. 6, June 1997, p 417-420), coupled with the use of additives such as polyethylene glycol (PEG), detergents, and other components such as hexamine cobalt, spermidine, and polyvinylpyrollidone (PVP). Such methods also disclosed by ferrreira etal (Trends in biotechnology, 380-388, 2000) on by Stadles et al (J. of Gene Medecine, S54-S66, 2004). However, currently known methods are unable to provide an efficient and cost effective separation of supercoiled and nicked (or relaxed) DNA. In addition, many of the known methods suffer from the disadvantage of using PEG or other additives, which may not be desired in manufacture of plasmid DNA, as they require additional separation, disposal and quality control methods, which can be difficult, more time consuming and more expensive. Alternative forms of known methods for separation of supercoiled and relaxed forms of plasmid DNA utilize very expensive resins, which also utilize solvents, such as acetonitrile, ethanol and other components, like triethylamine and tetrabutyl ammonium phosphate, during processing. Additional methods of separating supercoiled and relaxed DNA rely on size-exclusion chromatography, which involves separation of the two forms of plasmid DNA based on the small difference in size. These columns tend to be relatively long, posing significant scale-up problems, making it infeasible to implement in large-scale production. In addition size-exclusion methods need concentrated sample solutions that are infeasible to obtain with plasmid DNA solutions, due to the highly viscous nature of the DNA.

Also, plasmid DNA preparations, which are produced from bacterial preparations and often contain a mixture of relaxed and supercoiled plasmid DNA, often requires endotoxin removal, as required by the FDA, as endotoxins produced by many bacterial hosts are known to cause inflammatory reactions, such as fever or sepsis in the host receiving the plasmid DNA. These endotoxins are generally lipopolysaccharides, or fragments thereof, that are components of the outer membrane of Gram-negative bacteria, and are present in the DNA preparation of the host cells and host cell membranes or macromolecules. Hence removal of endotoxins is a crucial and necessary step in the purification of plasmid DNA for therapeutic or prophylactic use. Endotoxin removal from plasmid DNA solutions primarily used the negatively charged structure of the endotoxins. However plasmid DNA also is negatively charged and hence separation is usually achieved with anion exchange resins which bind both these molecules and, under certain conditions, preferentially elute plasmid DNA while binding the endotoxins. Such a separation results in only partial removal as significant amounts of endotoxins elute with the plasmid DNA and/or a very poor recovery of plasmid DNA is achieved.

Large scale isolation and purification of plasmid DNA from large volume microbial fermentations thus requires the development of an improved plasmid preparation process. Also a process for separating and purifying a large quantity of plasmids DNA in simpler way and in shorter time is required. It is also desirable for plasmid-based research and therapy, that the nucleic acids can be separated and purified keeping the same structure in a reproducible manner, and in order to avoid the adverse effect of impurities on mammalian body, the nucleic acids are required to have been separated and purified up to high purity.

With said conventional method, however, there is a problem that the nucleic acids, in particular, plasmids DNA cannot be obtained in sufficient high purity and in sufficient large quantity. Therefore, the present invention aims at providing a separating method that utilizes at least two chromatography steps, which enables to separate a large quantity of plasmids DNAs in a shorter time and with an unexpectedly high purity grade.

## SUMMARY OF THE INVENTION

The invention is based on the discovery of a method for producing and isolating highly purified plasmid DNA. The plasmid DNA produced and isolated by the method of the invention contains very low levels of contaminating chromosomal DNA, RNA, protein, and endotoxins. The plasmid DNA produced according to the invention is of sufficient purity for plasmid-based therapy.

<The present invention relates to a method of preparing a pharmaceutical grade plasmid DNA composition comprising providing a cell extract containing plasmid DNA, wherein the cells have been lysed through alkaline lysis and the cell membranes and genomic DNA has been removed by an initial extraction or filtration; and thereafter performing at least two chromatography steps comprising in the following order: anion exchange chromatography, triplex helix chromatography and hydrophobic interaction chromatography, wherein the composition prepared has less than about 0.0001 % host cell genomic DNA contamination.>

Additional objects and advantages of the invention will be set forth in part in the description which follows, and in part will be obvious from the description, or may be learned by practice of the invention. The objects and advantages of the invention will be realized and attained by means of the elements and combinations particularly pointed out in the

appended claims.

It is to be understood that both the foregoing general description and the following detailed description are exemplary and explanatory only and are not restrictive of the invention, as claimed.

The accompanying drawings, which are incorporated in and constitute a part of this specification, illustrate several embodiments of the invention and together with the description, serve to explain the principles of the invention.

## BRIEF DESCRIPTION OF THE FIGURES

**Figure 1** is a schematic of the apparatus that may be used for continuous mode cell lysis of the invention.

**Figure 2** is a schematic of the mixer M1 in the continuous cell lysis apparatus.

**Figure 3** is a table comparing purification yields in terms of gDNA, RNA, proteins, endotoxin contaminant using a single step of anion exchange chromatography (AEC), or a two-step method with a step of anion exchange chromatography in combination with triple helix affinity chromatography (THAC), and a three-step method comprising a step of anion exchange chromatography, a step triple helix affinity chromatography and a step of hydrophobic interaction chromatography (HIC) in combination ND means not detected : low sensitivity analytical methods.

**Figure 4** is a table comparing various methods of separating and purifying plasmid DNA, such anion-exchange chromatography (AEC), hydroxyapatite chromatography (HAC), hydrophobic interaction chromatography (HIC), reversed-phase chromatography (RPC), size exclusion chromatography (SEC), triple helix affinity chromatography (THAC) alone or in combination, and the method according to the present invention. Results in terms of quality of the purified plasmid DNA are provided herein. ND, not detected (low sensitivity analytical methods).

**Figures 5A and 5B** are graphs showing depurination and nicking rates (formation of open circular plasmid form) of the plasmid DNA stored at +25°C and +5°C for up to 90 days.

## Definitions

Acidic means relating to or containing an acid; having a pH of less than 7.

Alkaline means relating to or containing an alkali or base; having a pH greater than 7. Continuous means not interrupted, having no interruption.

Genomic DNA means a DNA that is derived from or existing in a chromosome.

Laminar flow means the type of flow in a stream of solution water in which each particle moves in a direction parallel to every particle.

Lysate means the material produced by the process of cell lysis. The term lysing refers to the action of rupturing the cell wall and/or cell membrane of a cell which is in a buffered solution (*i.e.*, cell suspension) through chemical treatment using a solution containing a lysing agent. Lysing agents include for example, alkali, detergents, organic solvents, and enzymes. In a preferred embodiment, the lysis of cells is done to release intact plasmids from host cells.

Neutralizes to make (a solution) neutral or to cause (an acid or base/alkali) to undergo neutralization. By this term we mean that something which neutralizes a solution brings the pH of the solution to a pH between 5 and 7, and preferably around 7 or more preferably closer to 7 than previously.

Newtonian fluid is a fluid in which shear stress is proportional to the velocity gradient and perpendicular to the plane of shear. The constant of proportionality is known as the viscosity. Examples of Newtonian fluids include liquids and gasses.

Non-Newtonian fluid is a fluid in which shear stress is not proportional solely to the velocity gradient and perpendicular to the plane of shear. Non-Newtonian fluids may not have a well defined viscosity. Non-Newtonian fluids include plastic solids, power-law fluids, viscoelastic fluids (having both viscous and elastic properties), and time-dependent viscosity fluids.

Plasmid DNA means a small cellular inclusion consisting of a ring of DNA that is not a chromosome, which may have the capability of having a non-endogenous DNA fragment inserted into it. As used herein, plasmid DNA can also be any form of plasmid DNA, such as cut, processed, or other manipulated form of a non-chromosomal DNA, including, for example, any of, or any combination of, nicked circle plasmid DNA, relaxed circle plasmid DNA, supercoiled plasmid DNA, cut plasmid DNA, linearized or linear plasmid DNA, and single-stranded plasmid DNA. Procedures for the construction of plasmids include those described in Maniatis et al., Molecular Cloning, A Laboratory Manual, 2d, Cold Spring Harbor Laboratory Press (1989). A protocol for a mini-prep of plasmid DNA, well-known in the art (Birnboim and Doly, Nucleic Acids Research 7:1513 (1979)), can be used to initially isolate plasmid DNA for later processing through some aspects of the invention and can be contrasted with the highly purified samples produced from the methods of the invention. Preferably, the form of the plasmid DNA is, or at least is after preparation by the purification method of the invention, substantially closed circular form plasmid DNA, or about 80%, 85%, 90%, 95%, or more than about 99%

closed circular form plasmid DNA. Alternatively, a supercoiled covalently closed form of pDNA (ccc) can be preferred in some therapeutic methods, where it may be more effective than the open-circular, linear, or multimeric forms. Therefore, the pharmaceutical grade plasmid DNA may be isolated from or separated from one or more fomrs of plasmid and substantially comprise one or more desired forms.

For purposes of the present invention the term flowing refers to the passing of a liquid at a particular flow rate (*e.g.*, liters per minute) through the mixer, usually by the action of a pump. It should be noted that the flow rate through the mixer is believed to affect the efficiency of lysis, precipitation and mixing.

The terms "nicked" and "relaxed" DNA means DNA that is not supercoiled. "Supercoiled" DNA is a term well understood in the art in describing a particular, isolated form of plasmid DNA. Other forms of plasmid DNA are also known in the art.

A "contaminating impurity" is any substance from which it is desired to separate, or isolate, DNA. Contaminating impurities include, but are not limited to, host cell proteins, endotoxin, host cell DNA, such as chromosomal DNA or genomic DNA, and/or host cell RNA. It is understood that what is or can be considered a contaminating impurity can depend on the context in which the methods of the invention are practiced. A "contaminating impurity" may or may not be host cell derived, *i.e.*, it may or may not be a host cell impurity.

"Isolating" or "purifying" a first component (such as DNA) means enrichment of the first component from other components with which the first component is initially found. Extents of desired and/or obtainable purification are provided herein.

The terms essentially free, and highly purified are defined as about 95% and preferably greater than 98.99% pure or free of contaminants, or possessing less than 5%, and preferably less than 1-2% contaminants.

Pharmaceutical grade DNA is defined herein as a DNA preparation that contains no more than about 5%, and preferably no more than about 1-2% of cellular components, such as cell membranes.

The invention further encompasses a method of producing and isolating highly purified plasmid DNA that is essentially free of contaminants and thus is pharmaceutical grade DNA. The plasmid DNA produced and isolated by the method of the invention contains very low levels, *i.e.,* part per millions (ppm) of contaminating chromosomal DNA, RNA, protein, and endotoxins, and contains mostly closed circular form plasmid DNA. The plasmid DNA produced according to the invention is of sufficient purity for use in research and plasmid-based therapy, and optionally for human clinical trial material and human gene therapy experiments and clinical trials.

A "pharmaceutical grade plasmid DNA composition" of the invention is one that is produced by a method of the invention and/or is a composition having at least one of the purity levels defined below as a "pharmaceutical grade plasmid DNA." Preferably, a "pharmaceutical grade plasmid DNA composition" of the invention is of a purity level defined by at least two of those identified below as a "pharmaceutical grade plasmid DNA," for example, less than about 0.00008% chromosomal or genomic DNA and less than about 0.00005% protein contaminant, or for example less than about 0.00008% chromosomal or genomic DNA and less than about 0.1 EU/mg endotoxins. Other combinations of purity levels are included under the definition. Of course, the pharmaceutical grade plasmid DNA composition can further comprise or contain added components desired for any particular use, including use in combination treatments, compositions, and therapies. The levels of chromosomal or genomic DNA, RNA, endotoxins or protein refers to contaminants from the cell-based production of plasmid or other contaminant(s) from the purification process.

As noted, "pharmaceutical grade plasmid DNA" is defined herein as a DNA preparation that contains on the level of one part per million or ppm (< 0.0001%, i.e. < 0.0001 mg per 100 mg of plasmid DNA) or less of genomic DNA, RNA, and/or protein contaminants.

Also or more precisely, "pharmaceutical grade plasmid DNA" herein can mean a DNA preparation that contains less than about 0.01%, or less than 0.001%, and preferably less than 0.0001%, or preferably less than 0.00008% (< 0.00008%, i.e. < 0.00008 mg per 100 mg of plasmid DNA) of chromosomal DNA or genomic DNA.

"Pharmaceutical grade plasmid DNA" can also mean a DNA preparation that contains less than about 0.01%, or less than 0.001%, and preferably less than 0.0001%, or preferably less than 0.00002% (< 0.00002%, i.e. < 0.00002 mg per 100 mg of plasmid DNA) of RNA contaminants.

"Pharmaceutical grade plasmid DNA" can also mean a DNA preparation that contains less than about 0.0001%, and most preferably less than 0.00005% (< 0.00005%, i.e. < 0.00005 mg per 100 mg of plasmid DNA) of protein contaminants.

"Pharmaceutical grade plasmid DNA" can also mean a DNA preparation that contains less than 0.1 EU/mg endotoxins.

The Pharmaceutical grade plasmid DNA means herein a DNA preparation that is preferably, predominantly circular in form, and more precisely DNA that contains more than 80%, 85%, 90%, 95%, or more than 99% of closed circular form plasmid DNA.

T tube refers to a T-shaped configuration of tubing, wherein a T-shape is formed by a single piece of tubing created in that configuration or more than one piece of tubing combined to create that configuration. The T tube has three arms and a center area where the arms join. A T tube may be used to mix ingredients as two fluids can flow each

into one of the arms of the T, join at the center area, and out the third arm. Mixing occurs as the fluids merge.

Turbulent flow means irregular random motion of fluid particles in directions transverse to the direction of the main flow, in which the velocity at a given point varies erratically in magnitude and direction.

Viscoelastic refers to fluids having both viscous and elastic properties.

## DETAILED DESCRIPTION OF THE INVENTION

The invention is based on the discovery of a scalable method for producing a high yield of pharmaceutical grade plasmid DNA. In particular, the invention is based on the discovery of a method for producing and isolating highly purified plasmid DNA using a continuous alkaline lysis of host cells.

As a first step host cells are inoculated, *i.e.* transformed with a plasmid DNA at exponential growth phase cells and streaked onto plates containing LB medium containing an antibiotic such as tetracycline. Single colonies from the plate are then inoculated each into 20 ml LB medium supplemented with the appropriate antibiotic tetracycline in separate sterile plastic Erlenmeyer flasks and grown for 12-16 hours at 37 °C in a shaking incubator. One of these cultures was then used to inoculate 200 ml of sterile LB medium supplemented in a 2 L Erlenmeyer flasks. This was grown at 37°C and 200 rpm in a shaking incubator and used to inoculate two 5 L Erlenmeyer flasks, and grown at 30 °C and 200 rpm in a shaking incubator and used to inoculate the fermenter vessel when in midexponential phase, after 5 hours and at an OD600 nm of 2 units.

Host cell cultures and inoculation are well known in the art. Generally, host cells are grown until they reach high biomass and cells are in exponential growth in order to have a large quantity of plasmid DNA. Two distinct methods may be employed, *i.e.*, batch and fed-batch fermentation.

Batch fermentation allows the growth rate to be controlled through manipulation of the growth temperature and the carbon source used. As used herein, the term "batch fermentation" is a cell culture process by which all the nutrients required for cell growth and for production of plasmid contained in the cultured cells are in the vessel in great excess (for example, 10-fold excess over prior art concentrations of nutrients) at the time of inoculation, thereby obviating the need to make additions to the sterile vessel after the post-sterilization additions, and the need for complex feeding models and strategies.

Another type of fermentation useful according to the invention is fed-batch fermentation, in which the cell growth rate is controlled by the addition of nutrients to the culture during cell growth. As used herein, "fed-batch fermentation" refers to a cell culture process in which the growth rate is controlled by carefully monitored additions of metabolites to the culture during fermentation. Fed-batch fermentation according to the invention permits the cell culture to reach a higher biomass than batch fermentation.

Examples of fermentation process and exemplary rates of feed addition are described below for a 50 L preparation. However, other volumes, for example 10 L, 50 L, or greater than 500 L, also may be processed using the exemplary feed rates described below, depending on the scale of the equipment.

Highly enriched batch medium and fed-batch medium fermentations are appropriate for the production of high cell density culture to maximize specific plasmid yield and allow harvest at high biomass while still in exponential growth.

Fed-batch Fermentation uses glucose or glycerol as a carbon source. The fermentation is run in batch mode until the initial carbon substrate (glucose) is exhausted. This point is noted by a sudden rise in DO and confirmed by glucose analysis of a sample taken immediately after this event. The previously primed feed medium pump is then started. The pump rate is determined by a model derived from Curless et al. (Bioeng. 38:1082-1090, 1991). The model is designed to facilitate control of the feed phase of a fed-batch process. In the initial batch process, a non-inhibitory concentration of substrate is consumed by cells growing at their maximum specific growth rate, giving a rapid rise in the biomass levels after inoculation. The culture cannot grow at this rate indefinitely due to the accumulation of toxic metabolites (Fieschio et al., "Fermentation Technology Using Recombinant Microorganisms." In Biotechnology, eds. H. J. Rhem and G. Reed. Weinheim: VCH Verlagsgesellschaft mbH 7b: 117-140, 1989). To allow continued logarithmic growth, the model calculates the time-based feed rate of the growth-limiting carbon substrate, without the need for feedback control, to give a fed-batch phase of growth at a set by the operator. This is chosen at a level which does not cause the build up of inhibitory catabolites and is sufficient to give high biomass.

Generally, batch fermentation uses high levels (e.g., 4-fold higher than prior art concentrations) of precursors are present in the enriched batch medium. In particular the quantities of yeast extract in the batch medium enriched form 5 g/l (as in LB medium) to 20 g/liter thus providing huge quantities of growth factors and nucleic acid precursors. The medium is also supplemented with ammonium sulfate (5 g/l) which acts as a source of organic nitrogen. The additions of precursors (organic nitrogen in the form of ammonium sulfate) during the feeding process in fed-batch fermentation are designed to prevent deleterious effects on plasmid quality.

One important aspect of the method according to the present invention is cell lysis. Thus, the present invention encompasses a process for producing and isolating highly purified plasmid DNA that includes the step of cells lysis in which there is (a) a means for turbulent flow to rapidly mix a cell suspension (solution 1 in Figure 1) with a solution that

lyses cells (solution 2 in Figure 1); and (b) a means for laminar flow to permit incubating a mixture formed in (a) without substantial agitation, wherein the mixture formed in (a) flows from the means for turbulent flow into the means for laminar flow.

According to one embodiment of the invention, the mechanism may additionally comprise a means for adding a second solution that neutralizes the lysing solution (solution 3 in Figure 1), wherein the mixture incubated in (b) flows from the means for laminar flow into the means for adding a second solution.

In yet another embodiment, the mechanism may be used in a method to isolate plasmid DNA from cells comprising: (a) mixing the cells with an alkali lysing solution in the means for turbulent flow; and (b) neutralizing the alkaline lysing solution by adding an acidic solution.

Despite the numerous methods currently used to lyse bacterial cells, none of them address the problems caused by the viscoelastic properties of the fluids and the shear forces involved during mixing steps. One object of the present invention is a method of using T tubes for mixing the cell suspension (solution 1) and the alkaline solution (solution 2) uniformly and very rapidly before the viscoelastic fluid appears. Thus continuous lysis according to the present invention provides a major advantage in limiting shear forces. T tubes have generally small diameter tubing, usually with a diameter inferior to 1cm, preferably of around 2 and 8 mm, and more preferably of around 6mm, in order to increase contact time of mixed fluids, but that method does not make use of mixing induced by passage through the tube. Table 1 herein below shows variation of parameters B1a, B1b, B2 of the means for turbulent flow, laminar flow, and turbulent flow, respectively, and their corresponding flow rates S1, S2, and S3 as displayed in Figure 1.

**Table 1**

| B1a (60L/h) | | B1b (60L/h) | | B2 (90L/h) | | Flow rates | |
|---|---|---|---|---|---|---|---|
| diameter | length | diameter | length | diameter | length | S1, S2 et S3 | Range |
| 5 to 7mm | 2-6 m | 12.5 to 19 mm | 13 to 23 m | 5 to 8 mm | 2 to 4 m | 60/60/90 L/h | ± 20% |

Another object of the present invention is a mixer or injector with tubes instead of a T, which permits dispersion of the cells into the lysis solution. Accordingly, the mechanical stress on the fluids that pass through the tubes is greatly reduced compared to when the fluids are stirred, *e.g.*, by paddles in tanks. The initial efficiency of mixing results in even greater efficiency in the seconds that follow, since this fluid does not yet have viscoelastic properties and the mixing realized by the small diameter tube is very efficient. In contrast, when a T tube is used for mixing, the initial mixing is only moderate while the fluid becomes rapidly viscoelastic, resulting in considerable problems while flowing in the tube. This partial mixing results in lysis of only a portion of the cells and therefore can only release a portion of the plasmids before neutralization.

According to the present invention, we have identified two phases during lysis, named Phase I and Phase II. These two phases correspond to I) lysis of the cells and II) denaturation of nucleic acids, causing a major change in rheological behavior that results in a viscoelastic fluid. Adjusting the diameters of the tubes makes it possible to meet the needs of these two phases. Within a small diameter tube (B1a), mixing is increased. This is the configuration used for Phase I. Within a large diameter tube (B1b), the mixing (and thus the shear stress) is reduced. This is the configuration employed for Phase II.

Accordingly, we use a mixer called M1 that is depicted in Figure 2. Any T shaped device may also be used to provide dispersion of the cell suspension according to the present invention. With this mixer, solution 1 is injected counter currently into the alkaline lysis solution through one or more small diameter orifices in order to obtain an efficient dispersion. Diameters of these orifices are around 0.5 mm to 2 mm, and preferably about 1 mm in the configuration depicted.

The mixture exits mixer M1 to pass through a tube of small diameter (Figure 1) for a short time period (of about 2.5 sec). Combination of the diameter and flow time may be easily calculated to maintain a turbulent flow. Examples of variations of these parameters are provided in Table 1. All references to tube diameter provide the inner diameter of the tube, not the outer diameter, which includes the thickness of the tube walls themselves. This brief residence time in the tube permits very rapid homogenization of solutions 1 and 2. Assuming that solution 1 and solution 2 are still Newtonian fluids during Phase I, the flow mode is turbulent during the homogenization phase. At the exit from this tube, solutions 1 and 2 are homogenized, and the lysis of cells in suspension starts.

The homogenized mixture then passes through a second tube (B1b) of much larger diameter (Figure 1), in which lysis of the cells and formation of the viscoelastic fluid occurs. During this phase, mixing may be minimized and the solution may be allowed to "rest" to limit turbulence as much as possible in order to minimize any shear stress that would otherwise fragment gDNA. In one embodiment of the present invention, a contact time of about 1 to 3 min, around 2 min, and preferably of 1 min 20 sec is sufficient to complete the cell lysis and to denature nucleic acids. During the denaturation phase, the flow mode of the fluid is laminar, promoting slow diffusion of SDS and sodium hydroxide toward cellular components.

The lysate thus obtained and the neutralization solution 3 is then mixed with a Y mixer called M2. In one embodiment of the present invention, the inside diameter of the Y mixer is around 4 to 15 min, or around 6 to 10 mm, and may be of around 6mm or around 10 mm. The small diameter tube (*e.g.*, about 6 mm tube) is positioned at the outlet of the Y mixer to allow for rapid (< 1 sec) and effective mixing of the lysate with solution 3. The neutralized solution is then collected in a harvesting tank. During neutralization, rapidly lowering the pH induces flocculate formation (*i.e.*, formation of lumps or masses). On the other hand, the partially denatured plasmid renatures very quickly and remains in solution. The flocs settle down gradually in the harvesting tank, carrying away the bulk of the contaminants.

The schematic drawing in Figure 1 shows one embodiment of the continuous lysis (CL) system. Continuous lysis may be used on its own or with additional processes.

The method of the present invention can be used to lyse any type of cell (*i.e.*, prokaryotic or eukaryotic) for any purpose related to lysing, such as releasing desired plasmid DNA from target cells to be subsequently purified. In a preferred embodiment, the method of the present invention is used to lyse host cells containing plasmids to release plasmids.

The process of continuous alkaline lysis step according to the present invention may be performed on cells harvested from a fermentation which has been grown to a biomass of cells that have not yet reached stationary phase, and are thus in exponential growth (2-10 g dry weight/liter). The continuous alkaline lysis step may also be performed on cells harvested from a fermentation which has been grown to a high biomass of cells and are not in exponential growth any longer, but have reached stationary phase, with a cellular concentration of approximately 10-200 g dry weight per liter, and preferably 12-60 g dry weight per liter.

Another important aspect of the invention is highly purified plasmid DNA compositions and pharmaceutical grade plasmid DNA compositions produced through a combination of chromatography steps, which may or may not be combined with the aforementioned cell lysis aspect. Thus, the invention further encompasses, or in addition comprises, a method of producing and isolating highly purified plasmid DNA that is essentially free of contaminants and thus is pharmaceutical grade DNA. The plasmid DNA produced and isolated by the method of the invention contains very low levels, *i.e.,* part per millions (ppm) of contaminating chromosomal DNA, RNA, protein, and endotoxins, and contains mostly closed circular form plasmid DNA. The plasmid DNA produced according to the invention is of sufficient purity for use research and plasmid-based therapy. As noted above, a pharmaceutical grade plasmid DNA composition of the invention can, in one aspect, be defined by a purity level with respect to one or more typical contaminants, such as host cell contaminants. Accordingly, a pharmaceutical grade plasmid DNA composition of the invention can be a composition that contains on the level of one part per million or ppm (< 0.0001%, i.e. < 0.0001 mg per 100 mg of plasmid DNA) or less of genomic DNA, RNA, and/or protein contaminants. More precisely, pharmaceutical grade plasmid DNA composition can comprise a plasmid DNA preparation that contains less than about 0.01%, or less than 0.001%, and preferably less than 0.0001%, or preferably less than 0.00008% (< 0.00008%, i.e. < 0.00008 mg per 100 mg of plasmid DNA) of host cell chromosomal DNA or genomic DNA. A pharmaceutical grade plasmid DNA composition can also comprise a plasmid DNA preparation that contains less than about 0.01%, or less than 0.001%, and preferably less than 0.0001%, or preferably less than 0.00002% (< 0.00002%, i.e. < 0.00002 mg per 100 mg of plasmid DNA) of host cell RNA contaminants. A pharmaceutical grade plasmid DNA composition can comprise a plasmid DNA preparation that contains less than about 0.0001 %, and most preferably less than 0.00005% (< 0.00005%, i.e. < 0.00005 mg per 100 mg of plasmid DNA) of host cell protein contaminants. A pharmaceutical grade plasmid DNA composition can also comprise a plasmid DNA preparation that contains less than 0.1 EU/mg endotoxins. In particular, any combination of at least two, or at least three, or four of these purity levels is also included in the invention. Thus, a composition having a detectable level of host cell genomic DNA of less than about 0.01 % and less than about 0.001 % host cell RNA can be included in the invention. Most preferably, the pharmaceutical grade plasmid DNA composition can have less than about 0.00008% host cell genomic DNA and less than about 0.00002% host cell RNA and less than about 0.00005% host cell protein. In fact, any combination of the purity levels noted above can be employed for any particular pharmaceutical grade plasmid DNA composition under the invention. The compositions can also comprise other pharmaceutically acceptable components, buffers, stabilizers, or compounds for improving gene transfer and particularly plasmid DNA transfer into a cell or organism.

In another aspect, the methods of the invention comprise the use of triple helix affinity chromatography, which is preceded by or followed by at least one additional chromatography technique, optionally or typically as the final purification steps or at least at the end or near the end of the plasmid purification scheme. In combination with triple helix affinity chromatography is preferably one or more of ion exchange chromatography, hydrophobic interaction chromatography, and gel permeation or size exclusion chromatography. Other techniques include hydroxyapatite (type I and II) chromatography, reversed phase, and affinity chromatography. Any available affinity chromatography protocol involving nucleic acid separation can be adapted for use. The anion exchange chromatography or any one or more of the other chromatography steps or techniques used can employ stationary phases, displacement chromatography methods, simulated moving bed technology, and/or continuous bed columns or systems. In addition, any one or more of the steps or techniques can employ high performance chromatography techniques or systems.

Thus, the method of the invention comprises purification steps including triple helix affinity chromatography

with a further step of ion exchange chromatography and further may include hydrophobic interaction chromatography or gel permeation chromatography. The step of ion exchange chromatography may be both in fluidized bed ion exchange chromatography and axial and/or radial high resolution anion exchange chromatography,

The method thus includes the alkaline lysis step described herein in combination with subsequent ion exchange chromatography, triple helix affinity chromatography and hydrophobic interaction chromatography steps, occurring in that order. A lysate filtration or other flocculate removal may precede the first chromatography step. Methods of the invention described herein for purifying plasmid DNA are scalable and thus amenable to scale-up to large-scale manufacture.

In some embodiments of the invention, continuous lysis may be combined with additional purification steps to result in a high purity product containing pDNA. It may, for example, be combined with at least one of flocculate removal (such as lysate filtration, settling, or centrifugation), ion exchange chromatography (such as cation or anion exchange), triplex affinity chromatography, and hydrophobic interaction chromatography. In one embodiment, continuous lysis is followed by anion exchange chromatography, triplex affinity chromatography, and hydrophobic interaction chromatography, in that order. In another embodiment, continuous lysis is followed by lysate filtration, anion exchange chromatography, triplex affinity chromatography, and hydrophobic interaction chromatography, in that order. These steps allow for a truly scaleable plasmid manufacturing process, which can produce large quantities of pDNA with unprecedented purity. Host DNA & RNA as well as proteins are in the sub-ppm range.

The method of the present invention may also use further steps of size exclusion chromatography (SEC), reversed-phase chromatography, hydroxyapatite chromatography, and/or other available chromatography techniques, methods, or systems in combination with the steps described herein in accordance with the present application.

A flocculate removal may be employed to provide higher purity to the resulting pDNA product. This step may be used to remove the bulk of precipitated material (flocculate). One mechanism of performing flocculate removal is through a lysate filtration step, such as through a 1 to 5 mm, and preferably a 3.5 mm grid filter, followed by a depth filtration as a polishing filtration step. Other methods of performing flocculate removal are through centrifugation or setlling.

Ion exchange chromatography may be employed to provide higher purity to the resulting pDNA product. Anion exchange may be selected depending on the properties of the contaminants and the pH of the solution.

Anion exchange chromatography may be employed to provide higher purity to the resulting pDNA product. Anion exchange chromatography functions by binding negatively charged (or acidic) molecules to a support which is positively charged. The use of ion-exchange chromatography, then, allows molecules to be separated based upon their charge. Families of molecules (acidics, basics and neutrals) can be easily separated by this technique. Stepwise elution schemes may be used, with many contaminants eluting in the early fractions and the pDNA eluted in the later fractions. Anion exchange is very efficient for removing protein and endotoxin from the pDNA preparation.

For the ion exchange chromatography, packing material and method of preparing such material as well as process for preparing, polymerizing and functionalizing anion exchange chromatography and eluting and separating plasmid DNA therethrough are well known in the art.

Compound to be used for the synthesis of base materials that are used for the packing material for anion exchange chromatography may be any compounds, if various functional groups that exhibit hydrophobicity or various ion exchange groups can be introduced by a post-reaction after the base materials are synthetized. Examples of monofunctional monomers include styrene, o-halomethylstyrene, m-halomethylstyrene, p-halomethylstyrene, o-haloalkylstyrene, m-haloalkylstyrene, p-haloalkylstyrene, α-methylstyrene, α-methyl-o-halomethylstyrene, α-methyl-m-halomethylstyrene, α-methyl-p-halomethylstyrene, α-methyl-o-haloalkylstyrene, α-methyl-m-haloalkylstyrene, α-methyl-p-haloalkylstyrene, o-hydroxymethylstyrene, m-hydroxymethylstyrene, p-hydroxymethylstyrene, o-hydroxyalkylstyrene, m-hydroxyalkylstyrene, p-hydroxylalkylstyrene, α-methyl-o-hydroxymethylstyrene, α-methyl-m-hydroxymethylstyrene, α-methyl-p-hydroxymethylstyrene, α-methyl-o-hydroxyalkylstyrene, α-methyl-m-hydroxyalkylstyrene, α-methyl-p-hydroxyalkylstyrene, glycidyl methacrylate, glycidyl acrylate, hydroxyethyl acrylate, hydroxymethacrylate, and vinyl acetate. Most preferred compounds are haloalkyl groups substituted on aromatic ring, halogens such as Cl, Br, I and F and straight chain and/or branched saturated hydrocarbons with carbon atoms of 2 to 15. Examples of polyfunctional monomers include divinylbenzene, trivinylbenzene, divinyltoluene, trivinyltoluene, divinylnaphthalene, trivinylnaphthalene, ethylene glycol dimethacrylate, ethylene glycol diacrylate, diethylene glycol dimethacrylate, diethylene glycol diacrylate, methylenebismethacrylamide, and methylenebisacrylamide.

Various ion exchange groups may be introduced by the post-reaction. Preparation of the base material includes a first step wherein monofunctional monomer and polyfunctional monomer are weighed out at an appropriate ratio and precisely weighed-out diluent or solvent which are used for the purpose of adjusting the pores in particles formed and similarly precisely weighed-out polymerization initiator are added, followed by well stirring. The mixture is then submitted to a oil-in-water type suspension polymerization wherein the mixture is added into an aqueous solution dissolved suspension stabilizer weighed out precisely beforehand, and oil droplets with aiming size are formed by mixing with stirrer, and polymerization is conducted by gradually warming mixed solution. Ratio of monofunctional monomer to polyfunctional monomer is generally around 1 mol of monofunctional monomer, and around 0.01 to 0.2 mol of polyfunctional monomer

so as to obtain soft particles of base material. A polymerization initiator is also not particularly restricted, and azobis type and/or peroxide type being used commonly are used.

Suspension stabilizers such as ionic surfactants, nonionic surfactants and polymers with amphipathic property or mixtures thereof may also be used to prevent the aggregation among oil droplets themselves.

The packing material to be used for ion exchange chromatography for purifying plasmid DNAs is preferable to have relatively large pore diameter, particularly within a range from 1500 to 4000 angstroms. Surface modification to introduce ion exchange groups to base materials is well known in the art.

Two types of eluents may be used for the ion exchange chromatography. A first eluent containing low-concentration of salt and a second eluent containing high-concentration of salt may be used. The eluting method consists in switching stepwise from the first eluent to the second eluent and the gradient eluting method continuously changing the composition from the first eluent to the second eluent. Buffers and salts that are generally used in these eluents for ion exchange chromatography may be used. For the first eluent containing low-concentration of salt, aqueous solution with concentration of buffer of 10 to 50 mM and pH value of 6 to 9 is particularly preferable. For the second eluent containing high-concentration of salt, aqueous solution with 0,1 to 2M sodium salt added to eluent C is particularly preferable. For the sodium salts, sodium chloride and sodium sulfate may be used.

In addition, a chelating agent for bivalent metal ion may be used such as for example, ethylenediamine-tetraacetic acid, for inhibiting the degradation of plasmids due to DNA-degrading enzymes in the lysate of Escherichia coli. The concentration of chelating agent for bivalent metal ion is preferably 0,1 to 100 mM.

A wide variety of commercially available anion exchange matrices are suitable for use in the present invention, including but not limited to those available from POROS Anion Exchange Resins, Qiagen, Toso Haas, Sterogene, Spherodex, Nucleopac, and Pharmacia. For example, the column (Poros II PI/M, 4.5 mm x 100) is initially equilibrated with 20 mM Bis/TRIS Propane at pH 7.5 and 0.7 M NaCl. The sample is loaded and washed with the same initial buffer. An elution gradient of 0.5 M to 0.85 M NaCl in about 25 column volumes is then applied and fractions are collected. Preferred anion exchange chromatography includes Fractogel TMAE HiCap.

According to a preferred embodiment of the process of separating and purifying plasmid DNA, the present invention relates to a method of separating and purifying nucleic acids and/or plasmid DNA by ion exchange chromatography and triple helix chromatography in combination for efficiently obtaining nucleic acids with high purity in large quantity.

Triplex helix affinity chromatography is described *inter alia* in the patents US 6,319,672, 6,287,762 as well as in international patent application published under WO02/77274 of the Applicant.

Triplex helix affinity chromatography is based on specific hybridization of oligonucleotides and a target sequence within the double-stranded DNA. These oligonucleotides may contain the following bases:

- thymidine (T), which is capable of forming triplets with A.T doublets of double-stranded DNA (Rajagopal et al., Biochem 28 (1989) 7859);
- adenine (A), which is capable of forming triplets with A.T doublets of double-stranded DNA;
- guanine (G), which is capable of forming triplets with G.C doublets of double-stranded DNA;
- protonated cytosine (C+), which is capable of forming triplets with G.C doublets of double-stranded DNA (Rajagopal et al., loc. cit.);
- uracil (U), which is capable of forming triplets with A.U or A.T base pairs.

Preferably, the oligonucleotide used comprises a cytosine-rich homopyrimidine sequence and the specific sequence present in the DNA is a homopurine-homopyrimidine sequence. The presence of cytosines makes it possible to have a triple helix which is stable at acid pH where the cytosines are protonated, and destabilized at alkaline pH where the cytosines are neutralized.

Oligonucleotide and the specific sequence present in the DNA are preferably complementary to allow formation of a triple helix. Best yields and the best selectivity may be obtained by using an oligonucleotide and a specific sequence which are fully complementary. For example, an oligonucleotide poly(CTT) and a specific sequence poly(GAA). Preferred oligonucleotides have a sequence 5'-GAGGCTTCTTCTTCTT CTTCTTCTT-3' (GAGG$(CTT)_7$ (SEQ ID NO: 1), in which the bases GAGG do not form a triple helix but enable the oligonucleotide to be spaced apart from the coupling arm; the sequence $(CTT)_7$. These oligonucleotides are capable of forming a triple helix with a specific sequence containing complementary units (GAA). The sequence in question can, in particular, be a region containing 7, 14 or 17 GAA units, as described in the examples.

Another sequence of specific interest is the sequence 5'-AAGGGAGGGAGGA GAGGAA-3' (SEQ ID NO: 2). This sequence forms a triple helix with the oligonucleotides 5'-AAGGAGAGGAGGGAGGGAA-3' (SEQ ID NO: 3) or 5'-TTGGTGTGGTGGGTGGGTT-3' (SEQ ID NO: 4). In this case, the oligonucleotide binds in an antiparallel orientation to the polypurine strand. These triple helices are stable only in the presence of $Mg^{2+}$ (Vasquez et al., Biochemistry, 1995, 34, 7243-7251; Beal and Dervan, Science, 1991, 251, 1360-1363).

As stated above, the specific sequence can be a sequence naturally present in the double-stranded DNA, or a synthetic sequence introduced artificially in the latter. It is especially advantageous to use an oligonucleotide capable of forming a triple helix with a sequence naturally present in the double-stranded DNA, for example in the origin of replication of a plasmid or in a marker gene. To this regard, it is known through sequence analyses that some regions of these DNAs, in particular in the origin of replication, could possess homopurine-homopyrimidine regions. The synthesis of oligonucleotides capable of forming triple helices with these natural homopurine-homopyrimidine regions advantageously enables the method of the invention to be applied to unmodified plasmids, in particular commercial plasmids of the pUC, pBR322, pSV, and the like, type. Among the homopurine-homopyrimidine sequences naturally present in a double-stranded DNA, a sequence comprising all or part of the sequence 5'-CTTCCCGAAGGGAGAAAGG-3' (SEQ ID NO: 5) present in the origin of replication of E. coli plasmid ColE1 may be mentioned. In this case, the oligonucleotide forming the triple helix possesses the sequence: 5'-GAAGGGCTTCCCTCTTTCC-3' (SEQ ID NO: 6), and binds alternately to the two strands of the double helix, as described by Beal and Dervan (J. Am. Chem. Soc. 1992, 114, 4976-4982) and Jayasena and Johnston (Nucleic Acids Res. 1992, 20, 5279-5288). The sequence 5'-GAAAAAGGAAGAG-3' (SEQ ID NO: 7) of the plasmid pBR322 β-lactamase gene (Duval-Valentin et al., Proc. Natl. Acad. Sci. USA, 1992, 89, 504-508) may also be mentioned.

Appropriate target sequences which can form triplex structures with particular oligonucleotides have been identified in origins of replication of plasmids ColE1 as well as plasmids pCOR. pCOR plasmids are plasmids with conditional origin of replication and are *inter alia* described US 2004/142452 and US 2003/161844. ColE1-derived plasmids contain a 12-mer homopurine sequence (5'-AGAAAAAAAGGA-3') (SEQ ID NO: 8) mapped upstream of the RNA-II transcript involved in plasmid replication (Lacatena et al., 1981, Nature, 294, 623). This sequence forms a stable triplex structure with the 12-mer complementary 5'-TCTTTTTTTCCT-3' (SEQ ID NO: 9) oligonucleotide. The pCOR backbone contains a homopurine stretch of 14 non repetitive bases (5'-AAGAAAAAAAAGAA-3') (SEQ ID NO: 10) located in the A+T-rich segment of the γ origin replicon of pCOR (Levchenko et al., 1996, Nucleic Acids Res., 24, 1936). This sequence forms a stable triplex structure with the 14-mer complementary oligonucleotide 5'-TTCTTTTTTTTCTT-3' (SEQ ID NO: 11). The corresponding oligonucleotides 5'-TCTTTTTTTCCT-3' (SEQ ID NO: 8) and 5'-TTCTTTTTTTTCTT-3' (SEQ ID NO:11) efficiently and specifically target their respective complementary sequences located within the origin of replication of either ColE1 ori or pCOR (oriγ). In fact, a single non-canonical triad (T*GC or C*AT) may result in complete destabilization of the triplex structure.

The use of an oligonucleotide capable of forming a triple helix with a sequence present in an origin of replication or a marker gene is especially advantageous, since it makes it possible, with the same oligonucleotide, to purify any DNA containing the said origin of replication or said marker gene. Hence it is not necessary to modify the plasmid or the double-stranded DNA in order to incorporate an artificial specific sequence in it.

Although fully complementary sequences are preferred, it is understood, however, that some mismatches may be tolerated between the sequence of the oligonucleotide and the sequence present in the DNA, provided they do not lead to too great a loss of affinity. The sequence 5'-AAAAAAGGGAATAAGGG-3' (SEQ ID NO: 12) present in the E. coli β-lactamase gene may be mentioned. In this case, the thymine interrupting the polypurine sequence may be recognized by a guanine of the third strand, thereby forming a G*TA triplet which it is stable when flanked by two T*AT triplets (Kiessling et al., Biochemistry, 1992, 31, 2829-2834).

According to a particular embodiment, the oligonucleotides of the invention comprise the sequence $(CCT)_n$, the sequence $(CT)_n$ or the sequence $(CTT)_n$, in which n is an integer between 1 and 15 inclusive. It is especially advantageous to use sequences of the type $(CT)_n$ or $(CTT)_n$. The Applicant showed, in effect, that the purification yield was influenced by the amount of C in the oligonucleotide. In particular, as shown in Example 7, the purification yield increases when the oligonucleotide contains fewer cytosines. It is understood that the oligonucleotides of the invention can also combine (CCT), (CT) or (CTT) units.

The oligonucleotide used may be natural (composed of unmodified natural bases) or chemically modified. In particular, the oligonucleotide may advantageously possess certain chemical modifications enabling its resistance to or its protection against nucleases, or its affinity for the specific sequence, to be increased. Oligonucleotide is also understood to mean any linked succession of nucleosides which has undergone a modification of the skeleton with the aim of making it more resistant to nucleases. Among possible modifications, oligonucleotide phosphorothioates, which are capable of forming triple helices with DNA (Xodo et al., Nucleic Acids Res., 1994, 22, 3322-3330), as well as oligonucleotides possessing formacetal or methylphosphonate skeletons (Matteucci et al., J. Am. Chem. Soc., 1991, 113, 7767-7768), may be mentioned. It is also possible to use oligonucleotides synthesized with α anomers of nucleotides, which also form triple helices with DNA (Le Doan et al., Nucleic Acids Res., 1987, 15, 7749-7760). Another modification of the skeleton is the phosphoramidate link. For example, the $N^{3'}$-$P^{5'}$ internucleotide phosphoramidate link described by Gryaznov and Chen, which gives oligonucleotides forming especially stable triple helices with DNA (J. Am. Chem. Soc., 1994, 116, 3143-3144), may be mentioned. Among other modifications of the skeleton, the use of ribonucleotides, of 2'-O-methylribose, phosphodiester, etc. (Sun and Hélène, Curr. Opinion Struct. Biol., 116, 3143-3144) may also be mentioned. Lastly, the phosphorus-based skeleton may be replaced by a polyamide skeleton as in PNAs (peptide nucleic

acids), which can also form triple helices (Nielsen et al., Science, 1991, 254, 1497-1500; Kim et al., J. Am. Chem. Soc., 1993, 115, 6477-6481), or by a guanidine-based skeleton, as in DNGs (deoxyribonucleic guanidine, Proc. Natl. Acad. Sci. USA, 1995, 92, 6097-6101), or by polycationic analogues of DNA, which also form triple helices.

The thymine of the third strand may also be replaced by a 5-bromouracil, which increases the affinity of the oligonucleotide for DNA (Povsic and Dervan, J. Am. Chem. Soc., 1989, 111, 3059-3061). The third strand may also contain unnatural bases, among which there may be mentioned 7-deaza-2'-deoxyxanthosine (Milligan et al., Nucleic Acids Res., 1993, 21, 327-333), 1-(2-deoxy-β-D-ribofuranosyl)-3-methyl-5-amino-1*H*-pyrazolo[4,3-*d*]pyrimidin-7-one (Koh and Dervan, J. Am. Chem. Soc., 1992, 114, 1470-1478), 8-oxoadenine, 2-aminopurine, 2'-O-methylpseudoisocytidine, or any other modification known to a person skilled in the art (for a review see Sun and Hélène, Curr. Opinion Struct. Biol., 1993, 3, 345-356).

Another type of modification of the oligonucleotide has the aim, more especially, of improving the interaction and/or affinity between the oligonucleotide and the specific sequence. In particular, a most advantageous modification according to the invention consists in methylating the cytosines of the oligonucleotide. The oligonucleotide thus methylated displays the noteworthy property of forming a stable triple helix with the specific sequence in pH ranges closer to neutrality (≥ 5). It hence makes it possible to work at higher pH values than the oligonucleotides of the prior art, that is to say at pH values where the risks of degradation of plasmid DNA are much smaller.

The length of the oligonucleotide used in the method of the invention is between 5 and 30. An oligonucleotide of length greater than 10 bases is advantageously used. The length may be adapted by a person skilled in the art for each individual case to suit the desired selectivity and stability of the interaction.

The oligonucleotides according to the invention may be synthesized by any known technique. In particular, they may be prepared by means of nucleic acid synthesizers. Any other method known to a person skilled in the art may quite obviously be used.

To permit its covalent coupling to the support, the oligonucleotide is generally functionalized. Thus, it may be modified by a thiol, amine or carboxyl terminal group at the 5' or 3' position. In particular, the addition of a thiol, amine or carboxyl group makes it possible, for example, to couple the oligonucleotide to a support bearing disulphide, maleimide, amine, carboxyl, ester, epoxide, cyanogen bromide or aldehyde functions. These couplings form by establishment of disulphide, thioether, ester, amide or amine links between the oligonucleotide and the support. Any other method known to a person skilled in the art may be used, such as bifunctional coupling reagents, for example.

Moreover, to improve the hybridization with the coupled oligonucleotide, it can be advantageous for the oligonucleotide to contain an "arm" and a "spacer" sequence of bases. The use of an arm makes it possible, in effect, to bind the oligonucleotide at a chosen distance from the support, enabling its conditions of interaction with the DNA to be improved. The arm advantageously consists of a linear carbon chain, comprising 1 to 18 and preferably 6 or 12 ($CH_2$) groups, and an amine which permits binding to the column. The arm is linked to a phosphate of the oligonucleotide or of a "spacer" composed of bases which do not interfere with the hybridization. Thus, the "spacer" can comprise purine bases. As an example, the "spacer" can comprise the sequence GAGG. The arm is advantageously composed of a linear carbon chain comprising 6 or 12 carbon atoms.

Triplex affinity chromatography is very efficient for removing RNA and genomic DNA. These can be functionalized chromatographic supports, in bulk or prepacked in a column, functionalized plastic surfaces or functionalized latex beads, magnetic or otherwise. Chromatographic supports are preferably used. As an example, the chromatographic supports capable of being used are agarose, acrylamide or dextran as well as their derivatives (such as Sephadex, Sepharose, Superose, etc.), polymers such as poly(styrene/divinylbenzene), or grafted or ungrafted silica, for example. The chromatography columns can operate in the diffusion or perfusion mode.

To obtain better purification yields, it is especially advantageous to use, on the plasmid, a sequence containing several positions of hybridization with the oligonucleotide. The presence of several hybridization positions promotes, in effect, the interactions between the said sequence and the oligonucleotide, which leads to an improvement in the purification yields. Thus, for an oligonucleotide containing n repeats of (CCT), (CT) or (CTT) motifs, it is preferable to use a DNA sequence containing at least n complementary motifs, and preferably n+ 1 complementary motif. A sequence carrying n+ 1 complementary motif thus affords two positions of hybridization with the oligonucleotide. Advantageously, the DNA sequence contains up to 11 hybridization positions, that is to say n+10 complementary motifs.

The method according to the present invention can be used to purify any type of double-stranded DNA. An example of the latter is circular DNA, such as a plasmid, generally carrying one or more genes of therapeutic importance. This plasmid may also carry an origin of replication, a marker gene, and the like. The method of the invention may be applied directly to a cell lysate. In this embodiment, the plasmid, amplified by transformation followed by cell culture, is purified directly after lysis of the cells. The method of the invention may also be applied to a clear lysate, that is to say to the supernatant obtained after neutralization and centrifugation of the cell lysate. It may quite obviously be applied also to a solution prepurified by known methods. This method also enables linear or circular DNA carrying a sequence of importance to be purified from a mixture comprising DNAs of different sequences. The method according to the invention can also be used for the purification of double-stranded DNA.

The cell lysate can be a lysate of prokaryotic or eukaryotic cells.

As regards prokaryotic cells, the bacteria E. coli, B. subtilis, S. typhimurium or Strepomyces may be mentioned as examples. As regards eukaryotic cells, animal cells, yeasts, fungi, and the like, may be mentioned, and more especially Kluyveromyces or Saccharomyces yeasts or COS, CHO, C127, NIH3T3, and the like, cells.

The method of the present invention which includes at least a step of triplex affinity chromatography may be employed to provide higher purity to the resulting pDNA product. In triplex affinity chromatography, an oligonucleotide is bound to a support, such as a chromatography resin or other matrix. The sample being purified is then mixed with the bound oligonucleotide, such as by applying the sample to a chromatography column containing the oligonucleotide bound to a chromatography resin. The desired plasmid in the sample will bind to the oligonucleotide, forming a triplex. The bonds between the oligonucleotide and the plasmid may be Hoogsteen bonds. This step may occur at a pH ≤5, at a high salt concentration for a contact time of 20 minutes or more. A washing step may be employed. Finally, cytosine deprotonation occurs in a neutral buffer, eluting the plasmid from the oligonucleotide-bound resin.

According to the most preferred embodiment, the process of separating and purifying nucleic acids and/or plasmid DNAs comprises the steps of ion exchange chromatography, triple helix affinity chromatography, and hydrophobic interaction chromatography in combination.

Hydrophobic interaction chromatography uses hydrophobic moieties on a substrate to attract hydrophobic regions in molecules in the sample for purification. It should be noted that these HIC supports work by a "clustering" effect; no covalent or ionic bonds are formed or shared when these molecules associate. Hydrophobic interaction chromatography is beneficial as it is very efficiently removes open circular plasmid forms and other contaminants, such as gDNA, RNA, and endotoxin.

Synthesis of base materials for hydrophobic interaction chromatography, as well as process for preparing, polymerizing and functionalizing hydrophobic interaction chromatography and eluting and separating plasmid DNA therethrough are well known in the art, and are *inter alia* described in US patent No: 6,441,160 .

Compound to be used for the synthesis of base materials that are used for the packing material for hydrophobic interaction chromatography may be any compounds, if various functional groups that exhibit hydrophobicity or various ion exchange groups can be introduced by a post-reaction after the base materials are synthetized. Examples of monofunctional monomers include styrene, o-halomethylstyrene, m-halomethylstyrene, p-halomethylstyrene, o-haloalkylstyrene, m-haloalkylstyrene, p-haloalkylstyrene, α-methylstyrene, α-methyl-o-halomethylstyrene, α-methyl-m-halomethylstyrene, α-methyl-p-halomethylstyrene, α-methyl-o-haloalkylstyrene, α-methyl-m-haloalkylstyrene, α-methyl-p-haloalkylstyrene, o-hydroxymethylstyrene, m-hydroxymethylstyrene, p-hydroxymethylstyrene, o-hydroxyalkylstyrene, m-hydroxyalkylstyrene, p-hydroxylalkylstyrene, α-methyl-o-hydroxymethylstyrene, α-methyl-m-hydroxymethylstyrene, α-methyl-p-hydroxymethylstyrene, α-methyl-o-hydroxyalkylstyrene, α-methyl-m-hydroxyalkylstyrene, α-methyl-p-hydroxyalkylstyrene, glycidyl methacrylate, glycidyl acrylate, hydroxyethyl acrylate, hydroxymethacrylate, and vinyl acetate. Most preferred compounds are haloalkyl groups substituted on aromatic ring, halogens such as Cl, Br, I and F and straight chain and/or branched saturated hydrocarbons with carbon atoms of 2 to 15.

Examples of polyfunctional monomers include divinylbenzene, trivinylbenzene, divinyltoluene, trivinyltoluene, divinylnaphthalene, trivinylnaphthalene, ethylene glycol dimethacrylate, ethylene glycol diacrylate, diethylene glycol dimethacrylate, diethylene glycol diacrylate, methylenebismethacrylamide, and methylenebisacrylamide.

Various hydrophobic functional groups or various ion exchange groups may be introduced by the post-reaction. In order to minimize the influence on aiming products desired to separate due to the hydrophobicity exhibited by the base material itself, or the swelling or shrinking of the base material itself due to the change in salt concentration and the change in pH value, the base material is preferably prepared using relatively hydrophilic monomers, such as glycidyl methacrylate, glycidyl acrylate, hydroxyethyl acrylate, hydroxymethacrylate, and vinyl acetate. Preparation of the base material includes a first step wherein monofunctional monomer and polyfunctional monomer are weighed out at an appropriate ratio and precisely weighed-out diluent or solvent which are used for the purpose of adjusting the pores in particles formed and similarly precisely weighed-out polymerization initiator are added, followed by well stirring. The mixture is then submitted to a oil-in-water type suspension polymerization wherein the mixture is added into an aqueous solution dissolved suspension stabilizer weighed out precisely beforehand, and oil droplets with aiming size are formed by mixing with stirrer, and polymerization is conducted by gradually warming mixed solution.

Ratio of monofunctional monomer to polyfunctional monomer is generally around 1 mol of monofunctional monomer, and around 0.01 to 0.2 mol of polyfunctional monomer so as to obtain soft particles of base material. The ration of polyfunctional monomer may be increased to around 0.2 to 0.5 mol so as to obtain hard particles of base materials. Polyfunctional monomer alone may be used to obtain ever harder particules.

A polymerization initiator is also not particularly restricted, and azobis type and/or peroxide type being used commonly are used.

Suspension stabilizers such as ionic surfactants, nonionic surfactants and polymers with amphipathic property or mixtures thereof may also be used to prevent the aggregation among oil droplets themselves.

The diameter of formed particles is generally around of 2 to 500 μm. Preferred diameter of the particles is

comprised between 2 to 30 μm, and more preferably around 2 to 10 μm. When aiming at large scale purification of nucleic acids with high purity, it is around 10 to 100 μm and, when separating the aiming product from crude stock solution, it may be 100 to 500 μm, more preferably around 200 to 400 μm. For adjusting the particle diameter, the rotational speed of stirrer may be adjusted during polymerization. When particles with small diameter are needed, the number of revolutions may be increased and, when large particles are desired, the number of revolutions may be decreased. Here, since the diluent to be used is used for adjusting pores in formed particles, the selection of diluent is particularly important. As a fundamental concept, for the solvent to be used for polymerization, adjustment is made by variously combining a solvent that is poor solvent for monomer with a solvent that is good solvent for monomer. The size of pore diameter may be selected appropriately depending on the molecular size of nucleic acids designed to separate, but it is preferable to be within a range of 500 to 4000 angstroms for the packing material for hydrophobic interaction chromatography and within a range from 1500 to 4000 angstroms for the packing material for ion exchange chromatography.

In the hydrophobic interaction chromatography, for separating nucleic acids with different hydrophobicity preferable by utilizing packing materials with different hydrophobicity, respectively, the surface modification of the base material is important.

Hydrophobic groups may be selected among long chain or branched, including saturated hydrocarbon groups or unsaturated hydrocarbon groups with carbon atoms of 2 to 20. Aromatic ring may also be contained in the hydrocarbon group.

Hydrophobic groups may also be selected among compounds having the following formula:

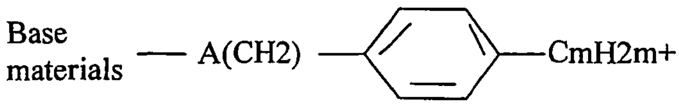

wherein n=0 to around 20 and the methylene group may be of straight chain or branched, m=0 to about 3 and hydrocarbon group may be of straight chain or branched, and A is C=O group or ether group, but methylene group may be bonded directly to base material without A.

Hydrophobic groups may further include ether group of alkylene glycol with carbon atoms of 2 to 20, which consists of repeating units of 0 to 10, wherein the opposite end of functional group reacted with base material may be OH group left as it is or may be capped with alkyl group with carbon atoms of 1 to 4.

The above described hydrophobic groups may be used solely or in mixture to modify the surface.

Chain of alkyl groups with carbon atoms of 6 to 20 carbon atoms are preferred for low hydrophobicity like plasmids. Long chain of alkyl groups having 2 to 15 carbon atoms for separating compounds with high hydrophobicity such as RNA originating from Escherichia coli and RNA in the cells of human and animals. Alkyl groups of 4 to 18 carbon atoms for separating compounds with relatively low hydrophobicity such as DNAs originating from Escherichia coli and DNAs in the cells of human and animals.

Upon separating these compounds, compounds may be selected appropriately to modify the surface without being confined to said exemplification. In effect, the degree of hydrophobicity of packing material varies depending on the concentration of salt in medium or the concentration of salt in eluent for adsorption. In addition the degree of hydrophobicity of packing material differs depending on the amount of the group introduced into the base material.

The pore diameter of the base material for hydrophobic interaction chromatography is particularly preferable to be 500 to 4000 angstroms, but it can be selected appropriately from said range depending on the molecular size of nucleic acids desired to separate. In general, since the retention of nucleic acids on the packing material and the adsorption capacity (sample leading) differ depending on the pore diameter, it is preferable to use a base material with large pore diameter for nucleic acids with large molecular size and a base material with small pore diameter for nucleic acids with small molecular size.

For example styrene base material may be reacted with hydrophobic group comprising long chain of alkyl groups, using halogen-containing compound and/or carbonyl halide and catalyst such as $FeCl_3$, $SnCl_2$ or $AlCl_3$, and utilizing Friedel-Craft reaction, it is possible to add directly to aromatic ring in base material as dehalogenated compound and/or acylated compound. In the case of the base material being particle containing halogen group, for example, using compounds with OH contained in functional group to be added, like butanol, and utilizing Williamson reaction with alkali catalyst such as NaOH or KOH, it is possible to introduce the functional group through ether bond. In the case of the functional group desired to add being amino group-containing compound, like hexylamine, it is possible to add using alkali catalyst such as NaOH or KOH and utilizing dehalogenic acid reaction. In the case of the base material containing OH group, inversely, if introducing epoxy group, halogen group or carbonyl halide group beforehand into the functional group desired to add, it is possible to introduce the functional group through ether or ester bond. In the case of the base material containing epoxy group, if reacting with compound with OH group or amino group contained in the functional

group desired to add, it is possible to introduce the functional group through ether or amino bond. Moreover, in the case of the functional group desired to add containing halogen group, it is possible to add the functional group through ether bond using acid catalyst. Since the proportion of functional group to be introduced into base material is influenced by the hydrophobicity of subject product desired to separate, it cannot be restricted, but, in general, packing material with around 0.05 to 4.0 mmol of functional group added per 1 g of dried base material is suitable.

With respect to the surface modification, a method of adding the functional group through post-reaction after formation of base material or particles is as described. Surface modification is conducted according to the same method, where the base material is formed after polymerization using monomers with said functional groups added before polymerization.

Base material may also be porous silica gel. A method of manufacturing silica gel, comprise silane coupling using a compound such as alkyltrimethoxysilane directly onto particles manufactured according to the method described in "Latest High-Speed Liquid Chromatography", page 289 ff. (written by Toshio Nambara and Nobuo Ikegawa, published by Tokyo Hirokawa Bookstore in 1988). Prior or after coupling the silane using epoxy group-containing silane coupling agent, a functional group may be added according to the method aforementioned. Proportion of functional group that is introduced around 0.05 to 4.0 mmol of functional group added per 1 g of dried base material is suitable.

Eluents are used in the hydrophobic interaction chromatography separation or purification step. Generally, two types of eluents are used. One eluent contains high-concentration of salt, while a second eluent contains low-concentration of salt. The eluting method comprises switching stepwise from an eluent having high concentration of salt to an eluent having a low concentration of salt and the gradient eluting method continuously changing the composition from one eluent to another may be used. For the buffers and salts generally used for the hydrophobic interaction chromatography can be used. For the eluent containing high-concentration of salt, aqueous solution with salt concentration of 1.0 to 4.5M and pH value of 6 to 8 is particularly preferable. For the eluent containing low-concentration of salt, aqueous solution with salt concentration of 0.01 to 0.5M and pH value of 6 to 8 is particularly preferable salts. Generally, ammonium sulfate and sodium sulfate may be used as salts.

The hydrophobic interaction chromatography plasmid DNA purification step may be conducted by combining a packing material introduced the functional group with weak hydrophobicity with a packing material introduced the functional group with strong hydrophobicity in sequence. In effect, medium cultured Escherichia coli contain in large quantity, various components different in hydrophobicity such as polysaccharides, Escherichia coli genome DNA, RNAs plasmids and proteins. It is also known that there are differences in the hydrophobicity even among nucleic acids themselves. Proteins that become impurities have higher hydrophobicity compared with plasmids.

Many hydrophobic interaction chromatography resins are available commercially, such as Fractogel propyl, Toyopearl, Source isopropyl, or any other resins having hydrophobic groups. Most preferred resins are Toyopearl bulk polymeric media. Toyopearl is a methacrylic polymer incorporating high mechanical and chemical stability. Resins are available as non-functionalized "HW" series resins and may be derivatized with surface chemistries for ion exchange chromatography or hydrophobic interactions. Four types of Toyopearl HIC resins featuring different surface chemistry and levels of hydrophobicity may be used. The hydrophobicity of Toyopearl HIC resins increases through the series: Ether, Phenyl, Butyl, and Hexyl. Structures of preferred Toyopearl HIC resins, i.e., Toyopearl HW-65 having 1000 angstroms pore diameter are showed below:

Toyopearl Ether-650

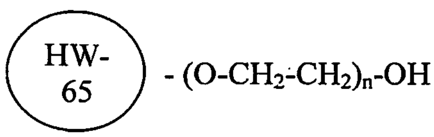

Toyopearl Phenyl-650

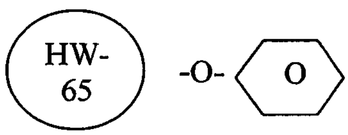

Toyopearl Butyl-650

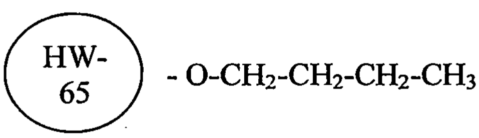

Toyopearl Hexyl-650 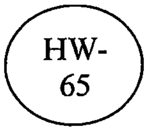 - $O-CH_2-CH_2-CH_2-CH_2-CH_2-CH_3$


The above described Toyopearl resins may have various particle size grade. Toyopearl 650C have a particle size of around 50 to 150 μm, preferably around 100μm, while Toyopearl 650M have a particle size of around 40 to 90μm, preferably around 65μm and Toyopearl 650S have a particle size of around 20 to 50 μm, preferably around 35μm. It is well known that particle size influences resolution, *i.e.*, resolution improves from C to M to S particle size grade, and thus increases with smaller particle sizes. Most preferred Toyopearl resin used in the HIC chromatography step within the process of separation and purification of the plasmid DNA according to the present invention is Toyopearl butyl-650S which is commercialized by Tosoh Bioscience.

According to a preferred embodiment, a further diafiltration step is performed. Standard, commercially available diafiltration materials are suitable for use in this process, according to standard techniques known in the art. A preferred diafiltration method is diafiltration using an ultrafiltration membrane having a molecular weight cutoff in the range of 30,000 to 500,000, depending on the plasmid size. This step of diafiltration allows for buffer exchange and concentration is then performed. The eluate is concentrated 3- to 4-fold by tangential flow filtration (membrane cut-off, 30 kDa) to a target concentration of about 2.5 to 3.0 mg/mL and the concentrate is buffer exchanged by diafiltration at constant volume with 10 volumes of saline and adjusted to the target plasmid concentration with saline. The plasmid DNA concentration is calculated from the absorbance at 260 nm of samples of concentrate. Plasmid DNA solution is filtered through a 0.2 μm capsule filter and divided into several aliquots, which are stored in containers in a cold room at 2-8°C until further processing. This yields a purified concentrate with a plasmid DNA concentration of supercoiled plasmid is around 70%, 75%, 80%, 85%, 90%, 95%, and preferably 99%. The overall plasmid recovery with this process is at least 35%, 40%, 45%, 50%, 55%, 60%, 65%, 70%, and 80%, with an average recovery of 60 %.

According to this embodiment, the diafiltration step is performed according the following conditions: buffer for step a) and for step b) are used:

i) a first diafiltration (step a) against 12.5 to 13.0 volumes of 50 mM Tris/HCl, 150 mM NaCl, pH 7.4 (named buffer I), and
ii) Perform a second diafiltration of the retentate from step a) above (step b) against 3.0 to 3.5 volumes of saline excipient (150 mM NaCl). This preferred diafiltration step according to the present invention efficiently and extensively removes ammonium sulfate and EDTA extensively. Also, subsequent to this diafiltration steps, appropriate physiological NaCl concentration (around 150mM) and final Tris concentration below 1 mM (between 200 μM and 1 mM) are obtained.

Plasmid DNA formulation so obtained by using this diafiltration step comprise NaCl as saline excipient and an appropriate concentration of Tris buffer so as to maintain or control the pH value between 7 and 7.5. Plasmid DNA formulations according to the present application are particularly useful as they plasmid DNA may surprisingly be stored in a stable non-degradable form in these conditions for prolonged period of time at 5°C and up to 25°C, that is at room temperature.

As described, according to the inventive method for separating plasmid DNA with high purity can be obtained in large quantity by simpler manipulation over conventional method.

The process of purifying plasmids may be used subsequently to the continuous lysis method as described above, or any alternative lysis methods which are well known in the art. For example, flow-through heat lysis of microbial cells containing plasmid may be used. This process is described *inter alia* in the International publication WO 96/02658. The particular heat exchanger consisted of a 10 ft. x 0.25 inch O.D. stainless steel tube shaped into a coil. The coil is completely immersed into a constant high temperature water bath. The hold-up volume of the coil is about 50 mL. Thermocouples and a thermometer were used to measure the inlet and exit temperatures, and the water bath temperature, respectively. The product stream is pumped into the heating coil using a Masterflex peristaltic pump with silicone tubing. Cell lysate exited the coil and is then centrifuged in a Beckman J-21 batch centrifuge for clarification. After centrifugation, the plasmid DNA may be purified using the method of purification according to the present invention.

Alternative cell lysis may make use of static mixers in series. In effect, as described in WO97/23601 , a first static mixer for lysing the cells through a first static mixer and for precipitating the cell lysate though a second static mixer may be used as an alternative method for lysing the cell prior to the method of purifying plasmid DNA according to the present invention. Large volumes of cells can be gently and continuously lysed in-line using the static mixer and that other static mixers are placed in-line to accomplish other functions such as dilution and precipitation. Suitable static mixers useful in the method of the present invention include any flow through device referred to in the art as a static or

motionless mixer of a length sufficient to allow the processes of the present invention. For example, for the purpose of lysing cells, the static mixer would need to have a length which would provide enough contact time between the lysing solution and the cells to 5 cause the lysis of the subject cells during, passage through the mixer. In a preferred embodiment, suitable static 5 mixers contain an internal helical structure which causes two liquids to come in contact with one another in an opposing rotational flow causing the liquids to mix together in a turbulent flow.

The method of separating and purifying plasmid DNA according to the present invention may be used to separate and purify any types of vectors with any sizes. The size range of plasmid DNA that may be separated by the method according to the present invention is from approximately 5 kb to approximately 50 kb, preferably 15 kb to 50 kb, which DNA includes a vector backbone of approximately 3 kb, a therapeutic gene and associated regulatory sequences. Thus, a vector backbone useful in the invention may be capable of carrying inserts of approximately 10-50 kb or larger. The insert may include DNA from any organism, but will preferably be of mammalian origin, and may include, in addition to a gene encoding a therapeutic protein, regulatory sequences such as promoters, poly adenylation sequences, enhancers, locus control regions, etc. The gene encoding a therapeutic protein may be of genomic origin, and therefore contain exons and introns as reflected in its genomic organization, or it may be derived from complementary DNA. Such vectors may include for example vector backbone replicatable with high copy number replication, having a polylinker for insertion of a therapeutic gene, a gene encoding a selectable marker, *e.g.*, SupPhe tRNA, the tetracycline kanamycin resistance gene, and is physically small and stable. The vector backbone of the plasmid advantageously permits inserts of fragments of mammalian, other eukaryotic, prokaryotic or viral DNA, and the resulting plasmid may be purified and used in vivo or ex vivo plasmid-based therapy. Vectors having relatively high copy number, *i.e.*, in the range of 20-40 copies/cell up to 1000-2000 copies/cell, may be separated and purified by the method according to the present invention. For example, a vector that includes the pUC origin of replication is preferred according to the method of the invention. The pUC origin of replication permits more efficient replication of plasmid DNA and results in a tenfold increase in plasmid copy number/cell over, e.g., a pBR322 origin. Preferably, plasmid DNA with conditional origin of replication or pCOR as described in US 2003/1618445, may be separated by the process according to the present invention. The resulting high copy number greatly increases the ratio of plasmid DNA to chromosomal DNA, RNA, cellular proteins and co-factors, improves plasmid yield, and facilitates easier downstream purification. Accordingly, any vector (plasmid DNA) may be used according to the invention. Representative vectors include but are not limited to NV1FGF plasmid. NV1FGF is a plasmid encoding an acidic Fibroblast Growth Factor or Fibroblast Growth Factor type 1 (FGF-1), useful for treating patients with end-stage peripheral arterial occlusive disease (PAOD) or with peripheral arterial disease (PAD). Camerota et al. (J Vase. Surg., 2002, 35, 5:930-936) describes that 51 patients with unreconstructible end-stage PAD, with pain at rest or tissue necrosis, have been intramuscularly injected with increasing single or repeated doses of NV1FGF into ischemic thigh and calf. Various parameters such as transcutaneous oxygen pressure, ankle and toe brachial indexes, pains assessment, and ulcer healing have been subsequently assessed. A significant increase of brachial indexes, reduction of pain, resolution of ulcer size, and an improved perfusion after NV1FGF administration are were observed.

Host cells useful according to the invention may be any bacterial strain, *i.e.,* both Gram positive and Gram negative strains, such as E. coli and Salmonella Typhimurium or Bacillus that is capable of maintaining a high copy number of the plasmids described above; for example 20-200 copies. E. coli host strains may be used according to the invention and include HB101, DH1, and DH5$\alpha$F, XAC-1 and XAC-1pir 116, TEX2, and TEX2pir42 (WO04/033664). Strains containing the F plasmid or F plasmid derivatives (for example JM109) are generally not preferred because the F plasmid may co-purify with the therapeutic plasmid product.

According to another aspect, the present invention also relates to composition comprising highly purified plasmid DNA that is essentially free of contaminants or in the range of sub-ppm contaminants and thus is pharmaceutical grade DNA. The pharmaceutically grade plasmid DNA composition according to the present invention thus contains sub-ppm (< 0.0001%, i.e. < 0.0001 mg per 100 mg of plasmid DNA) gDNA, RNA, and protein contaminants

The pharmaceutical grade plasmid DNA composition thus contains less than about 0.01%, or less than 0.001%, and preferably less than 0.0001%, or preferably less than 0.00008% (< 0.0008%, i.e. < 0.0008 mg per 100 mg of plasmid DNA) of chromosomal DNA or genomic DNA.

The pharmaceutical grade plasmid DNA composition thus contains less than about 0.01%, or less than 0.001%, and preferably less than 0.0001%, or preferably less than 0.00002% (< 0.0002%, i.e. < 0.0002 mg per 100 mg of plasmid DNA) of RNA contaminants.

The pharmaceutical grade plasmid DNA composition thus contains less than about 0.0001%, and most preferably less than 0.00005% protein contaminants.

The pharmaceutical grade plasmid DNA composition thus contains less than 0.1 EU/mg endotoxins.

The pharmaceutical grade plasmid DNA composition thus contains predominant circular in form, and more precisely contains more than 80%, 85%, 90%, 95%, or 99% of closed circular form plasmid DNA.

The present invention also relates to plasmid DNA liquid formulation that are stable and stays un-degraded at room temperature for long period of time, and are thus useful for storage of plasmid DNA that are used research and related human therapy.

The present invention thus relates to a stable plasmid DNA formulation comprising plasmid DNA, a very dilute buffer solution, and a saline excipient, wherein the buffer solution is present in a concentration so as to maintain the pH of said formulation or composition between 7 and 7.5.

Buffer solutions that are capable of maintaining the pH of the composition between 7 and 7.5 consist either of an acid/base system comprising Tris [tris(hydroxymethyl)-aminomethane], or lysine and an acid chosen from a strong acid (hydrochloric acid for example) or a weak acid (maleic acid, malic acid or acetic acid for example), or of an acid/ base system comprising Hepes [2-(4-(2-hydroxyethylpiperazin)-1-yl)ethanesulphonic acid] and a strong base (sodium hydroxide for example). The buffer solution may also comprise Tris/HCl, lysine/HCl, Tris/maleic acid, Tris/malic acid, Tris/acetic acid, or Hepes/sodium hydroxide.

Preferably, the pH is maintained between 7 and 7.5 and still more particularly at around 7.2.

Saline excipient that may be used in the formulation of the present invention is preferably NaCl at a concentration between 100 and 200 mM, and preferably a concentration of around 150mM. Other saline excipient may comprise anions and cations selected from the group consisting of acetate, phosphate, carbonate, $SO^{2-}_4$, $Cl^-$, $Br^-$, $NO_3^-$, $Mg^{2+}$, $Li^+$, $Na^+$, $K^+$, and $NH_4$+.

The molar concentration of the buffer solution is determined so as to exert the buffering effect within a limit and in a volume where the pH value is stabilized between 7 and 7.5. The stable plasmid-DNA storage composition according to the present invention thus comprises plasmid DNA, a saline excipient, and a buffer solution wherein the buffer solution is present in a concentration up to 1mM, and preferably between 250μM and 1mM, or preferably between 400μM and 1mM so as to maintain the pH of said formulation or composition between 7 and 7.5. Among the buffer systems according to the invention, the Tris buffer solution at a concentration of 400μM gives particularly satisfactory results and is thus preferred in the plasmid formulation of the present invention.

As shown in the Examples below, the plasmid DNA formulation according to the present invention exhibit an excellent stability both at 4°C and at room temperature (RT), *e.g.*, 20 or 25°C. Particularly, plasmid DNA formulation is useful for a prolonged period of time of 1 month, 2 months, 3 months, to 6 months and up to 12 months at 4°C and at 25°C, *e.g.,* RT.

The present invention thus relates to a composition comprising plasmid DNA, a buffer solution and saline excipient, wherein the buffer solution is present in a concentration sufficient to preserve plasmid DNA in stable form at 4°C to 25°C.

The present invention also relates to a composition comprising plasmid DNA, a buffer solution and saline excipient, wherein the buffer solution is present in a concentration sufficient to preserve plasmid DNA in stable form at 4°C to 25°C for a prolonged period of time, of 1 month, 2 months, 3 months, to 6 months and up to 12 months.

In effect, plasmid DNA that are stored at 5°C or at room temperature during long period of time exhibit very low depurination and open-circularization rates, inferior to 20%, 15%, 10%, 5%, or ≤ 1 % per month.

The composition according to the present invention may further comprise an adjuvant, such as for example a polymer selected among polyethylene glycol, a pluronic, or a polysorbate sugar, or alcohol.

According to another aspect, the present invention relates to a method of preserving plasmid DNA in a composition comprising a) preparing a purified sample of plasmid DNA and b) combining said purified sample of plasmid DNA with a saline excipient and a buffer solution that maintains the pH of the resulting composition between 7 and 7.5.

The present invention also relates to a method of preserving plasmid DNA in a composition at a temperature of up to about 20°C, comprising a) preparing a purified sample of plasmid DNA, b) combining the purified sample of plasmid DNA with a saline excipient and a buffer solution wherein the buffer solution is present in a concentration of less than 1mM, or between 250μM and 1mM, and preferably 400μM; and c) storing the plasmid DNA composition at a temperature of about 4°C up to about 20°C.

## Examples

### General techniques of cloning and molecular biology

The traditional methods of molecular biology, such as digestion with restriction enzymes, gel electrophoresis, transformation in E. coli, precipitation of nucleic acids and the like, are described in the literature (Maniatis et al., T., E.F. Fritsch, and J. Sambrook, 1989. Molecular cloning: a laboratory manual, second edition. Cold Spring Harbor Laboratory, Cold Spring Harbor Laboratory Press, New York; Ausubel F.M., R. Brent, R.E. Kinston, D.D. Moore, J.A. Smith, J.G. Seidman and K. Struhl. 1987. Current protocols in molecular biology 1987-1988. John Willey and Sons, New York.). Nucleotide sequences were determined by the chain termination method according to the protocol already published (Ausubel et al., 1987).

Restriction enzymes were supplied by New England Biolabs, Beverly, MA (Biolabs).

To carry out ligations, DNA fragments are incubated in a buffer comprising 50 mM Tris-HCl pH 7.4, 10 mM $MgCl_2$, 10 mM DTT, 2 mM ATP in the presence of phage T4 DNA ligase (Biolabs).

Oligonucleotides are synthesized using phosphoramidite chemistry with the phosphoramidites protected at the β position by a cyanoethyl group (Sinha, N.D., J. Biernat, J. McManus and H. Köster, 1984. Polymer support oligonucleotide synthesis, XVIII: Use of β-cyanoethyl-N,N-dialkylamino-/N-morpholino phosphoramidite of deoxynucleosides for the synthesis of DNA fragments simplifying deprotection and isolation of the final product. Nucl. Acids Res., 12, 4539-4557: Giles, J.W. 1985. Advances in automated DNA synthesis. Am. Biotechnol., Nov./Dec.) with a Biosearch 8600 automatic DNA synthesizer, using the manufacturer's recommendations.

Ligated DNAs or DNAs to be tested for their efficacy of transformation are used to transform the following strain rendered competent:

E. coli DH5α[F/endA1, hsdR17, supE44, thi-1, recA1, gyrA96, relA1, Δ(lacZYA-arqF)U169, deoR, Φ80dlac (lacZΔM15)] (for any Col E1 plasmid); or
E. coli XAC-pir (for any pCor-derived plasmid).

Minipreparations of plasmid DNA are made according to the protocol of Klein et al., 1980.

LB culture medium is used for the growth of E. coli strains (Maniatis et al., 1982). Strains are incubated at 37°C. Bacteria are plated out on dishes of LB medium supplemented with suitable antibiotics.

**Example 1**

The adjustment of the diameters to the flow rates used follows from calculation of Reynolds numbers in coils of the continuous lysis system. Because the following analysis assumes that the behavior of the fluids is Newtonian, the figures reported below are only fully valid in B1a and to a certain extent in B2.

The value of the Reynolds number allows one skilled in the art to specify the type of behavior encountered. Here, we will address only fluid flow in a tube (hydraulic engineering).

1) Non-Newtonian fluid

The two types of non-Newtonian fluids most commonly encountered in industry are Bingham and Ostwald de Waele.

In this case, the Reynolds number (Re) is calculated as follows:

$Re_N$ is the generalized Reynolds number

$$Re_N = (1 / (2^{n-3})) \times (n / 3n + 1)^n \times ((\rho \times D^n \times w^{2-n}) / m) \qquad (1)$$

D: inside diameter of the cross section (m)
p: volumetric mass of the fluid (kg/$m^3$)
w: spatial velocity of the fluid (m/s)
n: flow behavior index (dimensionless)
m: fluid consistency coefficient (dyn . $s^n$ / $cm^2$)

And n and m are determined empirically (study of rheological behavior).

2) Newtonian fluid

As for the first section, in Equation (1) we have:

Re = f(inside diameter, μ, ρ, and u) since n and m are functions of μ.

$$Re = (u \times D \times \rho) / \mu \qquad (2)$$

p: Volumetric mass of the fluid (kg/$m^3$)
μ: Viscosity of the fluid (Pa.s, and 1 mPa.s = 1 cP)
D: inside diameter of the cross section (m)
u: mean spatial velocity of the fluid (m/s)
Equation (1), for n=1, reduces to Equation (2).

With Q = flow rate ($m^3/h$) and S = surface area of the cross section ($m^2$) and if $\mu$ is given in cP, then:

$$Re = (4 \times (Q/3600) \times \rho) / ((\mu/1000) \times \Pi \times D) \qquad (3)$$

In a circular conduit, the flow is laminar for a Reynolds number below 2500, and is hydraulically smooth turbulent flow for a Reynolds number between 2000 and 500,000. The limit is deliberately vague between 2000 and 2500, where both types of behavior are used to determine what may then occur, and the choice is made *a posteriori.*

3) Calculations

Since n and m are generally not known, the following approximations have been used to estimate the trends:

Newtonian fluid (in all the cross sections)
$\rho$ = 1000 kg/$m^3$ (for all the fluids)
$\mu$ = 5 cP in B1a and 40 cP in B1b (our data)
2.5 cP in B2 (our data)

The following calculations were performed using Equation (3) for two standard tubing configurations tested called configuration 1 and configuration 2 (without B1b tube):

**Table 2**

| Coil | Configuration 1 | | Configuration 2 | |
|---|---|---|---|---|
| | B1a | B2 | B1a | B2 |
| Viscosity* (cP)<br>Diameter (mm)<br>Flow rate (L/h) | 5<br>12.7<br>60 | 2.5<br>9.5<br>105 | 5<br>6<br>12 | 2.5<br>6<br>21 |
| Reynolds number Process | 334<br>laminar | 1564<br>laminar | 141<br>laminar | 495<br>laminar |

In these two configurations, the flows are laminar at all stages and the solutions are not adequately mixed together.

For other tubing configurations (no B1b tube), we have:

**Table 3**

| Coil | High speed / std diameter | | High speed / 16 mm ID | | High speed / | 6 mm ID |
|---|---|---|---|---|---|---|
| | B1a | B2 | B1a | B2 | B1a | B2 |
| Viscosity* (cP)<br>Diameter (mm)<br>Flow rate (L/h) | 5<br>12<br>120 | 2.5<br>10<br>210 | 5<br>16<br>120 | 2.5<br>16<br>210 | 5<br>6<br>120 | 2.5<br>6<br>210 |
| Reynolds number Process | 707<br>laminar | 2971<br>turbulent | 531<br>laminar | 1857<br>laminar | 1415<br>laminar | 4951<br>turbulent |

Similar calculations were performed using Equation (3) for various tubing configurations with both B1a and B1b tubes present:

**Table 4**

| Coil | High speed | | | High speed / max agitation | | |
|---|---|---|---|---|---|---|
| | B1a | B1b | B2 | B1a | B1a | B1a |
| Viscosity* (cP)<br>Diameter (mm)<br>Flow rate (L/h) | 5<br>6<br>120 | 5<br>16<br>120 | 2.5<br>6<br>210 | 5<br>3<br>120 | 5<br>2<br>120 | 5<br>3<br>160 |

(continued)

| Coil | High speed | | | High speed / max agitation | | |
|---|---|---|---|---|---|---|
| | B1a | B1b | B2 | B1a | B1a | B1a |
| Reynolds number | 1415 | 531 | 4951 | 2829 | 4244 | 3773 |
| Process | laminar | laminar | turbulent | turbulent | turbulent | turbulent |

Clearly, predefined Reynolds values can be obtained by adjusting the tube diameters and the flow rates.

One skilled in the art can envision many combinations of diameters and lengths for B2 or for the two sections of B1 (B1a and B1b). For example, the first section of B1 can be reduced from 6 mm to 3 mm in order to reduce the length and increase the agitation. Additionally, n and m may be determined from the study of the rheological behavior of the fluids and used to determine the right characteristics of the tubes.

Besides the agitation efficiency, one may also consider the duration of the agitation, which in some embodiments of the present invention is obtained by adjusting the length of the coils.

The diameter of the tubes or the fluid velocity does not appear to dominate in Equation (1) for a non-Newtonian fluid (data not shown). In other words, it does not seem to be more effective to alter the diameter than it is to alter the flow rate if equation (1) is used for calculations within B1b and in B2. Where high flow rates are desirable, the diameter can be varied along with the flow rate.

These principles can be used as a basis for limiting agitation as much as possible in B1b and B2 in order to avoid fragmenting gDNA.

During lysis, agitation can be quite vigorous as long as gDNA is not denatured. Reducing the diameter at the beginning of B1 makes it possible to increase agitation (increased Re) in order to sufficiently mix solution 2 with the cells. On the other hand, when the cells are lysed, agitation and frictional forces against the wall may be reduced to avoid nucleic acid fragmentation. Increasing the diameter makes it possible to reduce agitation (decreased Re) and friction (lowered velocity).

M1: mixing the fluids.
B1a: fine-tuning the mixing at the beginning of lysis: convection phenomenon (macromixing).
B1b: letting denaturation occur plus diffusion phenomenon (micromixing).

It is assumed that the generalized Reynolds number has the same meaning for a non-Newtonian fluid as the classical Reynolds number has for a Newtonian fluid. In particular, it is assumed that the limit for the laminar regime in a conduit of circular cross section is $Re_N < 2300$.

Neutralization is performed within B2. High flow rates tend to increase the fragmentation of genomic DNA by causing agitation that is too vigorous and by frictional forces at the wall (mechanical stresses). Using a large diameter tube makes it possible to reduce agitation (Re) and frictional forces (velocity). We positioned here a small diameter tube (6 mm) to avoid having not enough agitation. Our observations show it is best having only a small diameter tube for B2, in order to "violently and quickly" agitate the neutralized lysate.

## Example 2

We can break down the CL system into 5 steps. In one particular embodiment, the configuration is as follows:

1) Mixing: cells (in solution 1) + solution 2 (M1 + 3 m of 6 mm tube). Beginning of lysis of the cells by SDS, no risk of fragmenting DNA as long as it is not denatured.
2) End of lysis and denaturation of gDNA (13 m of 16 mm tube).
3) Mixing: Lysate + solution 3 (M2 + 3 m of 6 mm tube).
4) Harvesting the neutralized lysate at 4°C
5) Settling down of flocs and large fragments of gDNA overnight at 4°C.

The following conditions may be used to carry out continuous lysis:

- Solution 1: EDTA 10 mM, glucose (G1c) 9 g/l and Tris HCl 25 mM, pH 7.2.
- Solution 2: SDS 1% and NaOH 0.2 N.
- Solution 3: Acetic acid 2 M and potassium acetate 3M.
- Flow rate 60 l/h: Solution 1 and solution 2
- Flow rate 90 1/h: Solution 3.

- Cells adjusted to 38.5 g/l with solution 1.

The cells in solution 1 pass through 3 nozzles that disperse them into solution 2, which arrives from the opposite direction.

- Mixer M1 has a geometry making it possible to optimize mixing of the two fluids (see Figure 2, schematic drawing of mixer).
- The first section of the tube after mixer M1 is B1a and the next section is B1b.

B1a: 3 m long, 6 mm diameter, 2.5 sec residence time
B1b: 13 m long, 16 mm diameter, 77 sec residence time

The process of the present invention provides an advantage in terms of efficiency, summarized as: dispersion, brief violent mixing, and gentle mixing by diffusion.
Using the process of the present invention, the number of cells lysed is increased and therefore the quantity of pDNA recovered is increased.
The idea of diffusion is especially important because of the difficulty of mixing these fluids due to their properties, in particular the viscoelasticity.
The process of the present invention makes it possible to limit shear stress and therefore to limit fragmentation of gDNA, facilitating its removal during subsequent chromatographic purification.
The problem is then mixing with solution 3, which may be cooled down to 4°C. In one embodiment, the process of the invention uses:

- Mixer M2, which is a Y of inside diameter of about 10 mm
- The section of the tube B2 placed after mixer M2.

B2: 2 m of 6 mm tube; residence time: 1 sec

Table 5 below gives the results obtained in comparative tests to show the advantages of our continuous lysis process compared to batch lysis.

**Table 5**

| | Ratio gDNA/pDNA in lysate | Quantity of plasmid extracted per g of cell (mg/g) |
|---|---|---|
| Batch lysis | 16.9 | 1.4 |
| Continuous lysis with CL system described in example 1 | 1.6 | 1.9 |

## Example 3

The column used is a 1 ml HiTrap column activated with NHS (N-hydroxysuccinimide, Pharmacia) connected to a peristaltic pump (output < 1 ml/min. The specific oligonucleotide used possesses an $NH_2$ group at the 5' end, its sequence is as follows:

5'-GAGGCTTCTTCTTCTTCTTCTTCTT-3' (SEQ ID NO: 1)

The buffers used in this example are the following:

Coupling buffer: 0.2 M $NaHCO_3$, 0.5 M NaCl, pH 8.3.
Buffer A: 0.5 M ethanolamine, 0.5 M NaCl, pH 8.3.
Buffer B: 0.1 M acetate, 0.5 M NaCl, pH 4.

The column is washed with 6 ml of 1 mM HCl, and the oligonucleotide diluted in the coupling buffer (50 nmol in 1 ml) is then applied to the column and left for 30 minutes at room temperature. The column is washed three times in succession with 6 ml of buffer A and then 6 ml of buffer B. The oligonucleotide is thus bound covalently to the column through a CONH link. The column is stored at 4°C in PBS, 0.1 % $NaN_3$, and may be used at least four times.

The following two oligonucleotides were synthesized: oligonucleotide 4817:

5'-GATCCGAAGAAGAAGAAGAAGAAGAAGAAGAAGAAGAAGAAGAAGAAGAA GAAGAAGG-3' (SEQ ID NO: 13) and oligonucleotide 4818 5'-AATTCCTTCTT CTTCTTCTTCTTCTTCTTCTTCTTCTTCTTCTTCTTCTTCTTCG-3' (SEQ ID NO: 14)

These oligonucleotides, when hybridized and cloned into a plasmid, introduce a homopurine-homopyrimidine sequence $(GAA)_{17}$ (SEQ ID NO: 15) into the corresponding plasmid, as described above.

The sequence corresponding to these two hybridized oligonucleotides is cloned at the multiple cloning site of plasmid pBKS+ (Stratagene Cloning System, La Jolla CA), which carries an ampicillin-resistance gene. To this end, the oligonucleotides are hybridized in the following manner: one μg of these two oligonucleotides are placed together in 40 ml of a final buffer comprising 50 mM Tris-HCl pH 7.4, 10 mM $MgCl_2$. This mixture is heated to 95°C and then placed at room temperature so that the temperature falls slowly. Ten ng of the mixture of hybridized oligonucleotides are ligated with 200 ng of plasmid pBKS+ (Stratagene Cloning System, La Jolla CA) digested with BamHI and EcoRI in 30 μl final. After ligation, an aliquot is transformed into DH5a. The transformation mixtures are plated out on L medium supplemented with ampicillin (50 mg/l) and X-gal (20 mg/l). The recombinant clones should display an absence of blue colouration on this medium, contrary to the parent plasmid (pBKS+) which permits α-complementation of fragment ω of E. coli β-galactosidase. After minipreparation of plasmid DNA from 6 clones, they all displayed the disappearance of the PstI site located between the EcoRI and BamHI sites of pBKS+, and an increase in molecular weight of the 448-bp PvuII band containing the multiple cloning site. One clone is selected and the corresponding plasmid designated pXL2563. The cloned sequence is verified by sequencing using primer -20 (5'-TGACCGGCAGCAAAATG-3' (SEQ ID NO: 16)) (Viera J. and J. Messing. 1982. The pUC plasmids, an M13mp7-derived system for insertion mutagenesis and sequencing with synthetic universal primers. Gene, 19, 259-268) for plasmid pBKS+ (Stratagene Cloning System, La Jolla CA). Plasmid pXL2563 is purified according to Wizard Megaprep kit (Promega Corp. Madison, WI) according to the supplier's recommendations. This plasmid DNA preparation is used in examples described below.

Plasmid pXL2563 is purified on the HiTrap column coupled to the oligonucleotide, described in 1.1., from a solution also containing plasmid pBKS+.
The buffers used in this purification are the following:

Buffer F: 2 M NaCl, 0.2 M acetate, pH 4.5 to 5.
Buffer E: 1 M Tris-HCl, pH 9, 0.5 mM EDTA.

The column is washed with 6 ml of buffer F, and the plasmids (20 μg of pXL2563 and 20 μg of pBKS+ in 400 μl of buffer F) are applied to the column and incubated for 2 hours at room temperature. The column is washed with 10 ml of buffer F and elution is then carried out with buffer E. The plasmids are detected after electrophoresis on 1 % agarose gel and ethidium bromide staining. The proportion of the plasmids in the solution is estimated by measuring their transforming activity on E. coli.

Starting from a mixture containing 30 % of pXL2563 and 70 % of pBKS+, a solution containing 100 % of pXL2563 is recovered at the column outlet. The purity, estimated by the OD ratio at 260 and 280 nm, rises from 1.9 to 2.5, which indicates that contaminating proteins are removed by this method.

## Example 4

Coupling of the oligonucleotide (5'-GAGGCTTCTTCTTCTTCTTCTTCTT-3' (SEQ ID NO: 1)) to the column is performed as described in Example 3. For the coupling, the oligonucleotide is modified at the 5' end with an amine group linked to the phosphate of the spacer by an arm containing 6 carbon atoms (Modified oligonucleotide Eurogentec SA, Belgium). Plasmid pXL2563 is purified using the Wizard Megaprep kit (Promega Corp., Madison, WI) according to the supplier's recommendations. The buffers used in this example are the following:

Buffer F: 0-2 M NaCl, 0.2 M acetate, pH 4.5 to 5.
Buffer E: 1 M Tris-HCl pH 9, 0.5 mM EDTA.

The column is washed with 6 ml of buffer F, and 100 μg of plasmid pXL2563 diluted in 400 μl of buffer F are then applied to the column and incubated for 2 hours at room temperature. The column is washed with 10 ml of buffer F and elution is then carried out with buffer E. The plasmid is quantified by measuring optical density at 260 nm.

In this example, binding is carried out in a buffer whose molarity with respect to NaCl varies from 0 to 2 M (buffer F). The purification yield decreases when the molarity of NaCl falls. The pH of the binding buffer can vary from 4.5 to 5, the purification yield being better at 4.5. It is also possible to use another elution buffer of basic pH: elution is

thus carried out with a buffer comprising 50 mM borate, pH 9, 0.5 mM EDTA.

Coupling the oligonucleotide (5'-GAGGCTTCTTCTTCTTCTTCTTCTT-3' (SEQ ID NO: 1) to the column is carried out as described in Example 3. Plasmid pXL2563 is purified using the Wizard Megaprep kit (Promega Corp., Madison, WI) according to the supplier's recommendations. The buffers used in this example are the following:

Buffer F: 0.1 M NaCl, 0.2 M acetate, pH 5.
Buffer E: 1 M Tris-HCl pH 9, 0.5 mM EDTA.

The column is washed with 6 ml of buffer F, and 100 μg of plasmid pXL2563 diluted in 400 μl of buffer F are then applied to the column and incubated for one hour at room temperature. The column is washed with 10 ml of buffer F and elution is then carried out with buffer E. The content of genomic or chromosomal E. coli DNA present in the plasmid samples before and after passage through the oligonucleotide column is measured. This genomic DNA is quantified by PCR using primers in the E. coli galK gene. According to the following protocol: The sequence of these primers is described by Debouck et al. (Nucleic Acids Res. 1985, 13, 1841-1853):

5'-CCG AAT TCT GGG GAC CAA AGC AGT TTC-3' (SEQ ID NO: 17)
and 5'-CCA AGC TTC ACT GTT CAC GAC GGG TGT-3' (SEQ ID NO: 18).

The reaction medium comprises, in 25 μl of PCR buffer (Promega France, Charbonnières): 1.5 mM $MgCl_2$; 0.2 mM dXTP (Pharmacia, Orsay); 0.5 μM primer; 20 U/ml Taq polymerase (Promega). The reaction is performed according to the sequence:

- 5 min at 95°C
- 30 cycles of 10 sec at 95°C

  30 sec at 60°C

  1 min at 78°C

- 10 min at 78°C.

The amplified DNA fragment 124 base pairs in length is separated by electrophoresis on 3 % agarose gel in the presence of SybrGreen I (Molecular Probes, Eugene, USA), and then quantified by reference to an Ultrapur genomic DNA series from E. coli strain B (Sigma, ref D4889).

## Example 5

This example describes plasmid DNA purification from a clear lysate of bacterial culture, on the so-called "miniprep" scale: 1.5 ml of an overnight culture of DH5α strains containing plasmid pXL2563 are centrifuged, and the pellet is resuspended in 100 μl of 50 mM glucose, 25 mM Tris-HCl, pH 8, 10 mM EDTA. 200 μl of 0.2 M NaOH, 1 % SDS are added, the tubes are inverted to mix, 150 μl of 3 M potassium acetate, pH 5 are then added and the tubes are inverted to mix. After centrifugation, the supernatant is recovered and loaded onto the oligonucleotide column obtained as described in Example 1. Binding, washes and elution are identical to those described in Example 3. Approximately 1 μg of plasmid is recovered from 1.5 ml of culture. The plasmid obtained, analysed by agarose gel electrophoresis and ethidium bromide staining, takes the form of a single band of "supercoiled" circular DNA. No trace of high molecular weight (chromosomal) DNA or of RNA is detectable in the plasmid purified by this method.

## Example 6

This example describes a plasmid DNA purification experiment carried out under the same conditions as Example 5, starting from 20 ml of bacterial culture of DH5α strains containing plasmid pXL2563. The cell pellet is taken up in 1.5 ml of 50 mM glucose, 25 mM Tris-HCl, pH 8, 10 mM EDTA. Lysis is carried out with 2 ml of 0.2 M NaOH, 1 % SDS, and neutralization with 1.5 ml of 3 M potassium acetate, pH 5. The DNA is then precipitated with 3 ml of 2-propanol, and the pellet is taken up in 0.5 ml of 0.2 M sodium acetate, pH 5, 0.1 M NaCl and loaded onto the oligonucleotide column obtained as described in the above Example. Binding, washing of the column and elution are carried out as described in the above Example, except for the washing buffer, the molarity of which with respect to NaCl is 0.1M. The plasmid obtained, analysed by agarose gel electrophoresis and ethidium bromide staining, takes the form of a single band of "supercoiled" circular DNA. No trace of high molecular weight (chromosomal) DNA or of RNA is detectable in

the purified plasmid. Digestion of the plasmid with a restriction enzyme gives a single band at the expected molecular weight of 3 kilobases. The plasmid contains a cassette containing the cytomegalovirus promoter, the gene coding for luciferase and the homopurine-homopyrimidine sequence $(GAA)_{17}$ (SEQ ID NO: 15) originating from plasmid pXL2563. The strain DH1 (Maniatis et al., 1989) containing this plasmid is cultured in a 7-litre fermenter. A clear lysate is prepared from 200 grams of cells: the cell pellet is taken up in 2 litres of 25 mM Tris, pH 6.8, 50 mM glucose, 10 mM EDTA, to which 2 litres of 0.2 M NaOH, 1 % SDS, are added. The lysate is neutralized by adding one litre of 3M potassium acetate. After diafiltration, 4 ml of this lysate are applied to a 5 ml HiTrap-NHS column coupled to the oligonucleotide of sequence 5'-GAGGCTTCTTCTTCTTCTTCTTCTT-3' (SEQ ID NO: 1), according to the method described in Example 3. Washing and elution are carried out as described in the above Example.

## Example 7

This example describes the use of an oligonucleotide bearing methylated cytosines. The sequence of the oligonucleotide used is as follows:

5'-GAGG$^{Me}$CTT$^{Me}$CTT$^{Me}$CTT$^{Me}$CTT$^{Me}$CCT$^{Me}$CTT$^{Me}$CTT-3' (SEQ ID NO: 19)

This oligonucleotide possesses an $NH_2$ group at the 5' end. $^{Me}$C = 5-methylcytosine. This oligonucleotide enables plasmid pXL2563 to be purified under the conditions of Example 1 with a binding buffer of pH 5 (the risk of degradation of the plasmid is thereby decreased).

## Example 8

In the above examples, the oligonucleotide used is modified at the 5'-terminal end with an amine group linked to the phosphate through an arm containing 6 carbon atoms: $NH_2$-$(CH_2)_6$. In this example, the amine group is linked to the phosphate of the 5'-terminal end through an arm containing 12 carbon atoms: $NH_2$-$(CH_2)_{12}$. Coupling of the oligonucleotide and passage through the column are carried out as described in Example 3 with a buffer F: 2 M NaCl, 0.2 M acetate, pH 4.5. This oligonucleotide makes it possible to have better purification yields: a 53 % yield is obtained, whereas, with the oligonucleotide containing 6 carbon atoms, this yield is of the order of 45 % under the same conditions.

## Example 9

Following the cloning strategy described in Example 3, another two plasmids carrying homopurine-homopyrimidine sequences are constructed: the plasmid pXL2725 which contains the sequence $(GGA)_{16}$, (SEQ ID NO: 20) and the plasmid pXL2726 which contains the sequence $(GA)_{25}$ (SEQ ID NO: 21).

Plasmids pXL2725 and pXL2726, analogous to plasmid pXL2563, are constructed according to the cloning strategy described in Example 3, using the following oligonucleotide pairs:

5986: 5'-GATCC$(GA)_{25}$GGG-3' (SEQ ID NO: 22)
5987: 5'-AATTCCC$(TC)_{25}$G-3' (SEQ ID NO: 23)
5981: 5'-GATCC$(GGA)_{17}$GG-3' (SEQ ID NO: 24)
5982: 5'-AATT$(CCT)_{17}$CCG-3' (SEQ ID NO: 25)

The oligonucleotide pair 5986 and 5987 is used to construct plasmid pXL2726 by cloning the oligonucleotides at the BamHI and EcoRI sites of pBKS+ (Stratagene Cloning System, La Jolla CA), while the oligonucleotides 5981 and 5982 are used for the construction of plasmid pXL2725. The same experimental conditions as for the construction of plasmid pXL2563 are used, and only the oligonucleotide pairs are changed. Similarly, the cloned sequences are verified by sequencing on the plasmids. This enabled it to be seen that plasmid pXL2725 possesses a modification relative to the expected sequence: instead of the sequence GGA repeated 17 times, there is GGAGA$(GGA)_{15}$ (SEQ ID NO: 26).

## Example 10

The oligonucleotides forming triple helices with these homopurine sequences are coupled to HiTrap columns according to the technique described in Example 1.1. The oligonucleotide of sequence 5'-AATGCCTCCTCCTCCTCCTCCTCCT-3' (SEQ ID NO: 27) is used for the purification of plasmid pXL2725, and the oligonucleotide of sequence 5'-AGTGCTCTCTCTCTCTCTCTCTCTCT-3' (SEQ ID NO: 28) is used for the purification of plasmid pXL2726.

The two columns thereby obtained enabled the corresponding plasmids to be purified according to the technique

described in Example 2, with the following buffers:

Buffer F: 2 M NaCl, 0.2 M acetate, pH 4.5.
Buffer E: 1 M Tris-HCl, pH 9, 0.5 mM EDTA.

The yields obtained are 23 % and 31 % for pXL2725 and pXL2726, respectively.

**Example 11**

This example illustrates the influence of the length of the specific sequence present in the plasmid on the purification yields.
The reporter gene used in these experiments to demonstrate the activity of the compositions of the invention is the gene coding for luciferase (Luc).
The plasmid pXL2621 contains a cassette containing the 661-bp cytomegalovirus (CMV) promoter cloned upstream of the gene coding for luciferase, at the MluI and HindIII sites, into the vector pGL basic Vector (Promega Corp., Madison, WI). This plasmid is constructed using standard techniques of molecular biology.
The plasmids pXL2727-1 and pXL2727-2 are constructed in the following manner:
Two micrograms of plasmid pXL2621 were linearized with BamHI; the enzyme was inactivated by treatment for 10 min at 65°C; at the same time, the oligonucleotides 6006 and 6008 are hybridized as described for the construction of plasmid pXL2563.

6006: 5'-GATCT$(GAA)_{17}$CTGCAGATCT-3' (SEQ ID NO: 29)
6008: 5'-GATCAGATCTGCAG$(TTC)_{17}$A-3' (SEQ ID NO: 30).

This hybridization mixture is cloned at the BamHI ends of plasmid pXL2621 and, after transformation into DH5$\alpha$, recombinant clones are identified by PstI enzymatic restriction analysis, since the oligonucleotides introduce a PstI site. Two clones are selected, and the nucleotide sequence of the cloned fragment is verified using the primer (6282, 5'-ACAGTCATAAGTGCGGCGACG-3' (SEQ ID NO: 31)) as a sequencing reaction primer (Viera J. and J. Messing, 1982). The pUC plasmids an M13mp7-derived system for insertion mutagenesis and sequencing with synthetic universal primers. (Gene 19:259-268).
The first clone (pXL2727-1) contains the sequence GAA repeated 10 times. The second (pXL2727-2) contains the sequence 5'-GAAGAAGAG$(GAA)_7$GGAAGAGAA-3' (SEQ ID NO: 32).
A column such as the one described in Example 3, and which is coupled to the oligonucleotide 5'-GAGGCTTCTTCTTCTTCTTCTTCTT-3' (SEQ ID NO: 1), is used.
The plasmid pXL2727-1 carries 14 repeats of the sequence GAA. The oligonucleotide described above, which contains only 7 repeats of the corresponding hybridization sequence CTT, can hence hybridize with the plasmid at 8 different positions. Plasmid pXL2727-2, in contrast, possesses a hybridizing sequence $(GAA)_7$ (SEQ ID NO: 36) of the same length as that of the oligonucleotide bound to the column. This oligonucleotide can hence hybridize at only one position on pXL2727-2.
The experiment is identical to the one described in Example 4, with the following buffers:

Buffer F: 2 M NaCl, 0.2 M acetate, pH 4.5.
Buffer E: I M Tris-HCl, pH 9, 0.5 mM EDTA.

The purification yield is 29 % with plasmid pXL2727-1 and 19 % with pXL2727-2.
The cells used are NIH 3T3 cells, inoculated on the day before the experiment into 24-well culture plates on the basis of 50,000 cells/well. The plasmid is diluted in 150 mM NaCl and mixed with the lipofectant RPR115335. A lipofectant positive charges/DNA negative charges ratio equal to 6 is used. The mixture is vortexed, left for ten minutes at room temperature, diluted in medium without foetal calf serum and then added to the cells in the proportion of 1 $\mu$g of DNA per culture well. After two hours at 37°C, 10 % volume/volume of foetal calf serum is added and the cells are incubated for 48 hours at 37°C in the presence of 5 % of CO2. The cells are washed twice with PBS and the luciferase activity is measured according to the protocol described (Promega kit, Promega Corp. Madison, WI) on a Lumat LB9501 luminometer (EG and G Berthold, Evry). Plasmid pXL2727-1, purified as described in Example 8.2, gives transfection yields twice as large as those obtained with the same plasmid purified using the Wizard Megaprep kit (Promega Corp. Madison, WI).

## Example 12

The following example demonstrates the purification of pCOR-derived plasmids using triple-helix affinity chromatography. This technology has been shown to remove nucleic acid contaminants (particularly host genomic DNA and RNA) down to levels that have not been achieved with conventional chromatography methods.

A triplex affinity gel is synthesized with Sephacryl S-1000 SF (Amersham-Pharmacia Biotech) as the chromatography matrix. Sephacryl S-1000 is first activated with sodium *m*-periodate (3 mM, room temperature, 1 h) in 0.2 M sodium acetate (pH 4.7). Then the oligonucleotide is coupled through its 5'-$NH_2$ terminal moiety to aldehyde groups of the activated matrix by reductive amination in the presence of ascorbic acid (5 mM) as described previously for the coupling of proteins (Hornsey et al., J. Immunol. Methods, 1986, 93, 83-88). The homopyrimidine oligonucleotide used for these experiments (from Eurogentec, HPLC-purified) had a sequence which is complementary to a short 14-mer homopurine sequence (5'-AAGAAAAAAAAGAA-3') (SEQ ID NO: 10) present in the origin of replication (oriγ) of the pCOR plasmid (Soubrier et al., Gene Therapy, 1999, 6, 1482-1488). As discussed above, the sequence of the homopyrimidine oligonucleotide is 5'-TTCTTTTTTTTCTT-3' (SEQ ID NO: 11).

The following plasmids are chromatographed: pXL3296 (pCOR with no transgene, 2.0 kpb), pXL3179 (pCOR-FGF, 2.4 kpb), pXL3579 (pCOR-VEGFB, 2.5 kbp), pXL3678 (pCOR-AFP, 3.7 kbp), pXL3227 (pCOR-lacZ 5.4 kbp) and pXL3397 (pCOR-Bdeleted FVIII, 6.6 kbp). All these plasmids are purified by two anion-exchange chromatography steps from clear lysates obtained as described in example 4. Plasmid pBKS+ (pBluescript II KS + from Stratagene), a ColE1-derived plasmid, purified by ultracentrifugation in CsCl is also studied. All plasmids used are in their supercoiled (> 95 %) topological state or form.

In each plasmid DNA purification experiment, 300 μg of plasmid DNA in 6 ml of 2 M NaCl, 0.2 M potassium acetate (pH 5.0) is loaded at a flow rate of 30 cm/h on an affinity column containing the above-mentioned oligonucleotide 5'-TTCTTTTTTTTCTT-3' (SEQ ID NO: 11). After washing the column with 5 volumes of the same buffer, bound plasmid is eluted with 1 M Tris/HCl, 0.5 mM EDTA (pH 9.0) and quantitated by UV (260 nm) and ion-exchange chromatography with a Millipore Gen-Pak column (Marquet et al., BioPharm, 1995, 8, 26-37). Plasmid recoveries in the fraction collected are 207 μg for pXL3296, 196 μg for pXL3179, 192 μg for pXL3579, 139 μg for pXL3678, 97 μg for pXL 3227, and 79 μg for pXL 3397.

No plasmid binding could be detected (< 3 μg) when pBKS is chromatographed onto this column. This indicates that oligonucleotide 5'-TTCTTTTTTTTCTT-3' (SEQ ID NO: 11) makes stable triplex structures with the complementary 14-mer sequence 5'-AAGAAAAAAAAGAA-3' (SEQ ID NO: 10) present in pCOR (oriγ), but not with the closely related sequence 5'-AGAAAAAAAGGA-3' (SEQ ID NO: 8) present in pBKS. This indicates that the introduction of a single non-canonical triad (T*GC in this case) results in a complete destabilization of the triplex structure.

As a control, no plasmid binding (< 1 μg) was observed when pXL3179 is chromatographed on a blank column synthesized under strictly similar conditions but without oligonucleotide.

By operating this affinity purification column in the conditions reported here, the level of contamination by host genomic DNA was reduced from 2.6 % down to 0.07 % for a preparation of pXL3296. Similarly the level of contamination by host DNA is reduced from 0.5 % down to 0.008 % for a preparation of pXL3179 when the sample is chromatographed through the same affinity column.

## Example 13

The following example demonstrates the purification of ColEl-derived plasmids using triple-helix affinity chromatography. This technology has been shown to remove nucleic acid contaminants (particularly host genomic DNA and RNA) down to levels that have not been achieved with conventional chromatography methods.

A triplex affinity gel is synthesized by coupling of an oligonucleotide having the sequence 5'-TCTTTTTTTCCT-3' (SEQ ID NO: 9) onto periodate-oxidized Sephacryl S-1000 SF as described in the above Example.

Plasmids pXL3296 (pCOR with no transgene) and pBKS, a ColE1-derived plasmid, are chromatographed on a 1-ml column containing oligonucleotide 5'-TCTTTTTTTCCT-3' (SEQ ID NO: 9) in conditions described in Example 9. Plasmid recoveries in the fraction collected are 175 μg for pBKS and <1 μg for pXL3296. This indicates that oligonucleotide 5'-TCTTTTTTTCCT-3' (SEQ ID NO: 9) makes stable triplex structures with the complementary 12-mer sequence (5'-AGAAAAAAAGGA-3') (SEQ ID NO: 8) present in pBKS, but not with the very closely related 12-mer sequence (5'-AGAAAAAAAAGA-3') (SEQ ID NO: 34) present in pCOR. This indicates that the introduction of a single non-canonical triad (C*AT in this case) may result in complete destabilization of the triplex structure.

## Example 14

A seed culture is produced in an unbaffled Erlenmeyer flask by the following method. The working cell bank is inoculated into an Erlenmeyer flask containing M9modG5 medium, at a seed rate of 0.2%v/v. The strain is cultivated at

220 rpm in a rotary shaker at 37° ± 1°C for about 18 ± 2 hours until glucose exhaustion. This results in a 200 ml seed culture. The optical density of the culture is expected to be $A_{600}$ around 2-3.

A pre-culture in a first fermentor is then created. The seed culture is aseptically transferred to a pre-fermentor containing M9modG5 medium to ensure a seed rate of 0.2% (v/v) and cultivated under aeration and stirring. The $pO_2$ is maintained above 40% of saturation. The culture is harvested when the glucose is consumed after 16 hours. This results in about 30 liters of pre-culture. The optical density of the culture is expected to be $A_{600}$ around 2-3.

A main culture is then created in a second fermentor. 30 liters of preculture are aseptically transferred to a fermentor filled with 270 liters of sterilized FmodG2 medium to ensure a seed rate of about 10% (v/v). The culture is started on a batch mode to build some biomass. Glucose feeding is started once the initial sugar is consumed after about 4 hours. Aeration, stirring, $pO_2$ (40%), pH (6.9 ± 0.1), temperature (37±1°C) and glucose feeding are controlled in order to maintain a specific growth rate close to $0.09h^{-1}$. The culture is ended after about 35 hours of feeding. This results in about 400 liters of culture. The optical density of the culture is expected to be $A_{600}$ of about 100.

A first separation step is performed, which is called cell harvest. The biomass is harvested with a disk stack centrifuge. The broth is concentrated 3- to 4-fold to eliminate the spent culture medium and continuously resuspended in 400 liters of sterile S1 buffer. This results in about 500 liters of preconditioned biomass. DCW = 25 ± 5 g/L.

A second separation step is performed, which is called a concentration step. After resuspension/homogenization in S1 buffer, the cells are processed again with the separator to yield concentrated slurry. This results in about 60-80 liters of washed and concentrated slurry. DCW = 150 ± 30 g/L ; pDNA = 300 ± 60 mg/L.

A freezing step is then performed. The slurry is aseptically dispatched into 20-L Flexboy™ bags (filled to 50% of their capacity) and subsequently frozen at 20° ± 5°C before further downstream processing. This results in a frozen biomass. pDNA = 300 ± 60 mg/L ; supercoiled form > 95 % .

A cell thawing step is then performed. The frozen bags are warmed up to 20°C and the cell slurry is diluted to 40 g/L, pH 8.0 with 100 mM Tris hydrochloride, 10 mM EDTA, 20 mM glucose and the suspension is left at 20 ± 2 °C for 1 h under agitation before cell lysis. This results in thawed biomass slurry. pH=8.0 ± 0.2.

Temperatures around 20°C may be used during this step.

An alkaline lysis step is then performed. The cell lysis step is comprised of pumping the diluted cell suspension via an in-line mixer with a solution of 0.2 N NaOH-35 mM SDS (solution S2), followed by a continuous contact step in a coiled tubing. The continuous contact step is to ensure complete cell lysis and denaturation of genomic DNA and proteins. The solution of lysed cells is mixed in-line with solution 3 (S3) of chilled 3 M potassium acetate-2 N acetic acid, before collection in a chilled agitated vessel. The addition of solution S3 results in the precipitation of a genomic DNA, RNA, proteins and KDS.

A lysate filtration is performed next. The neutralized lysate is then incubated at 5 ± 3°C for 2 to 24 h without agitation and filtered through a 3.5 mm grid filter to remove the bulk of precipitated material (floc phase) followed by a depth filtration as polishing filtration step. This results in a clarified lysate, with a concentration of supercoiled plasmid of more than 90%.

Anion exchange chromatography is then performed. The clear lysate solution is diluted with purified water to a target conductivity value of 50 mS/cm, filtered through a double-layer filter (3 μm-0.8 μm) and loaded onto an anion-exchange chromatography column. A 300-mm column packed with 11.0 L Fractogel® TMAE HiCap (M) resin (Merck; #1.10316.5000) is used. The clear lysate is loaded onto the column and elution is performed using a step gradient of NaCl. The bulk of contaminants bound to the column are eluted with a NaCl solution at about 61 mS/cm, and DNA plasmid is eluted with a NaCl solution at about 72 mS/cm. This results in an ion exchange chromatography eluate having a high concentration of plasmid DNA.

This is followed by triplex affinity chromatography. The eluate from the anion exchange chromatography column is diluted with about 0.5 volumes of a solution of 500 mM sodium acetate (pH 4.2) containing 4.8 M NaCl and pumped through a triplex affinity chromatography column equilibrated with 50 mM sodium acetate (pH 4.5) containing 2 M NaCl. The column is 300 mm in diameter and contains 10.0 L of THAC Sephacryl™ S-1000 gel (Amersham Biosciences; Piscataway, NJ). The column is washed with a solution of 50 mM sodium acetate (pH 4.5) containing 1 M NaCl and NV1FGF is eluted with 100 mM Tris (pH 9.0) containing 0.5 mM EDTA. This results in a triplex affinity chromatography eluate having a high plasmid concentration.

A hydrophobic interaction chromatography step follows. The eluate of the affinity chromatography column is diluted with 3.6 volumes of a solution of 3.8 M ammonium sulfate in Tris (pH 8.0). After filtration through a 0.45 μm filter, the filtrate is loaded at 60 cm/h onto a hydrophobic interaction column (diameter 300 mm) packed with 9.0 L of Toyopearl® Butyl-650S resin (TosoH corp., Grove City, OH). The column is washed with a solution of ammonium sulfate at about 240 mS/cm and NV1FGF is eluted with ammonium sulfate at 220 mS/cm. This results in an HIC eluate free of relaxed forms.

According to a preferred embodiment, a further diafiltration step is performed. Standard, commercially available diafiltration materials are suitable for use in this process, according to standard techniques known in the art. A preferred diafiltration method is diafiltration using an ultrafiltration membrane having a molecular weight cutoff in the range of

30,000 to 500,000, depending on the plasmid size. This step of diafiltration allows for buffer exchange and concentration is then performed. The eluate of step 12 is concentrated 3- to 4-fold by tangential flow filtration (membrane cut-off, 30 kDa) to a target concentration of about 2.5 to 3.0 mg/mL and the concentrate is buffer exchanged by diafiltration at constant volume with 10 volumes of saline and adjusted to the target plasmid concentration with saline. The NV1FGF concentration is calculated from the absorbance at 260 nm of samples of concentrate. NV1FGF solution is filtered through a 0.2 μm capsule filter and stored in containers in a cold room at 2-8°C until further processing. This yields a purified concentrate with a plasmid DNA concentration of supercoiled plasmid is around 70%, 75%, 80%, 85%, 90%, 95%, and preferably 99%. The overall plasmid recovery with this process is at least 35%, 40%, 45%, 50%, 55%, 60%, 65%, 70%, and 80%, with an average recovery of 60 %.

## Example 15

The method of the above Example comprising an ion-exchange chromatography (AEC) step, a triple helix affinity chromatography step (THAC), and a hydrophobic chromatography step (HIC) results in a more purified plasmid DNA preparation are compared with previously known methods. This new method has been compared to previously known methods and has resulted in pDNA preparations having much lower amounts of genomic DNA, RNA, protein, and endotoxin. This is reflected in Figure 3. These experiments show that AEC, THAC and HIC provide a surprisingly higher purification yield comparing with some of the 2-step combinations for the effective removal of all contaminants. Combination of these steps provide a clear synergy in terms of efficacy of separation of plasmid DNA from other biological materials and contaminants, such as protein and endotoxin, RNA and genomic DNA, as well as open circular plasmid. In addition, the synergistic steps combination, i.e., AEC/THAC/HIC according to the present invention enables not only to obtain highly purified pharmaceutically grade plasmid DNA, but also compositions of highly pure and fully supercoiled, of more than 80%, 85%, 90%, 95% and more than 99% plasmid DNA.

## Example 16

The method of the above Example, which comprises an ion-exchange chromatography step, a triple helix affinity chromatography step, and a hydrophobic chromatography step for the preparation of highly purified plasmid DNA preparation is compared to previously known methods. As shown in Figure 4, the method according to the present invention surprisingly results in pDNA preparations having much lower amounts of genomic DNA, RNA, protein, and endotoxin, in the range of the sub-ppm. Also, as shown in Figure 4, the process of the present invention shows a product quality obtained at up to 10g.

## Example 17

The diafiltration step as described in Example 14 is performed according the following conditions: buffer for step a and for step b were used to determine the best conditions for:

iii) a first diafiltration (step a) against 12.5 to 13.0 volumes of 50 mM Tris/HCl, 150 mM NaCl, pH 7.4 (named buffer I), and

iv) Perform a second diafiltration of the retentate from step a) above (step b) against 3.0 to 3.5 volumes of saline excipient (150 mM NaCl).

This alternative diafiltration step according to the present invention efficiently and extensively removes ammonium sulfate and EDTA extensively. Also, subsequent to this diafiltration steps, appropriate target NaCl concentration around 150 mM and final Tris concentration between 400 μM and 1 mM are obtained. Examples of plasmid DNA formulations compositions are provided in the Table 6 below, and

**Table 6**

| Species | Final concentration | | |
|---|---|---|---|
| | 1st diafiltration | 2nd diafiltration | Active Pharmaceutical Ingredient |
| Ammonium sulfate | 10 μM | < 1 μM | < 1 μM |
| EDTA | 4 μM | < 1 μM | < 1 μM |
| Tris | 50 mM | 1.48 mM | 740 μM |
| NaCl | 154 mM | 154 mM | 154 mM |

**Example 18**

A technical batch of plasmid DNA NV1FGF API (active pharmaceutical ingredients) named LS06 is manufactured according to Example 13 with the diafiltration process step described in Example 17. The eluate is first diafiltered at around 2 mg API /mL against about 13 volumes of buffer I and the resulting retentate was diafiltered against about 3 volumes of saline excipient. The final retentate was then filtered through a 0.2 μm filter and adjusted to 1 mg/mL. The final API (pH 7.24) was stored in a Duran glass bottle at +5°C until DP manufacturing.

A stability study was performed on samples of LS06 stored in Duran glass bottles (API) as well as in 8-mL vials used for Drug Product manufacturing. After 90 days at +5°C the extent of both depurination and open-circularization for all samples was hardly detectable (≤ 0.3 %). After 90 days at +25°C the depurination and the open-circularization rates of LS06 samples were also quite low. The depurination and open-circularization rates calculated from this study were ≤ 1 % per month (Fig 8).

This study demonstrated that the stability profile of plasmid DNA NV1FGF is very stable in the formulation of the present invention wherein the pH values is maintain at around 7 to 7.5. While the depurination rate and plasmid nicking rates are generally strongly accelerated at +25°C, the Applicant has showed that the plasmid DNA stay stable in an non-degraded form for a long period of time even at RT.

Unless otherwise indicated, all numbers expressing quantities of ingredients, reaction conditions, and so forth used in the specification, including claims, are to be understood as being modified in all instances by the term "about." Accordingly, unless otherwise indicated to the contrary, the numerical parameters are approximations and may vary depending upon the desired properties sought to be obtained by the present invention. At the very least, and not as an attempt to limit the application of the doctrine of equivalents to the scope of the claims, each numerical parameter should be construed in light of the number of significant digits and ordinary rounding approaches.

Unless otherwise indicated, the term "at least" preceding a series of elements is to be understood to refer to every element in the series.

**REFERENCES**

**Patents:**

WO 98/00815 (utilization ofT [Tee], Pasteur Mérieux Sérums et Vaccins [Sera and Vaccines])

WO 96/02658, A.L. Lee et al., *A Method for Large Scale Plasmid Purification* (1996).

WO 97/23601, N.C. Wan et al., *Method for Lysing Cells* (1997).

WO 99/29832, D.S. McNeilly, *Method for purifying plasmid DNA and plasmid DNA substantially free of genomic DNA* (1999).

U.S. Patent No. 6,214,568, D.S. McNeilly *Method for purifying plasmid DNA and plasmid DNA substantially free of genomic DNA* (2001).

**Publications:**

H.C. Birnboim and J. Doly, A rapid alkaline extraction procedure for screening recombinant plasmid DNA Nucleic Acid Research 7(6):1513-1523 (1979).

D. Stephenson, F. Norman and R.H. Cumming, Shear thickening of DNA in SDS lysates Bioseparation 3:285-289 (1993).

M.S. Levy, L.A.S. Ciccolini, S.S.S. Yim, J.T. Tsai, N. Titchener-Hooker, P. Ayazi Shamlou and P. Dunnill, The effects of material properties and fluid flow intensity on plasmid DNA recovery during cell lysis Chemical Engineering Science 54:3171-3178 (1999).

## Claims

1. A method of preparing a pharmaceutical grade plasmid DNA composition comprising providing a cell extract containing plasmid DNA, wherein the cells have been lysed through alkaline lysis and the cell membranes and genomic DNA have been removed by an initial extraction or filtration; and thereafter performing at least three chromatography steps comprising in the following order: anion exchange chromatography, triple helix affinity chromatography, and hydrophobic interaction chromatography, wherein the composition prepared has less than about 0.0001 % host cell genomic DNA contamination.

2. The method of claim 1, wherein the first chromatography step performed is preceded by a lysate filtration.

3. The method of claim 1, wherein the first chromatography step performed is preceded by flocculate removal.

4. The method of claim 1, wherein the composition has less than about 0.0001 % host cell RNA contaminant.

5. The method of claim 1, wherein the composition has less than about 0.0001 % host cell protein contaminant.

6. The method of claim 4, wherein the composition has less than about 0.0001 % host cell protein contamination.

7. The method of claim 1, wherein the composition has less than about 0.1 EU/mg endotoxin.

8. The method of claim 6, wherein the composition has less than about 0.1 EU/mg endotoxin.

9. The method of claim 1, wherein the composition has less than or about 0.1 EU/mg endotoxin, less than or about 0.00008 % host cell protein contaminant, less than or about 0.00008 % host cell RNA contaminant, and less than or about 0.00008 % host cell genomic DNA contaminant.

10. The method of claim 1, wherein the composition has less than or about 0.00005 % host cell genomic DNA contaminant.

11. The method of claim 1, wherein the composition has less than or about 0.00008 % host cell genomic DNA contaminant.

12. The method of claim 1, wherein the composition has less than or about 0.1 EU/mg endotoxin, and less than or about 0.00005 % host cell protein contaminant.

13. The method of claim 12, wherein the composition has less than or about 0.00002 % host cell RNA contaminant, and less than or about 0.00008 % host cell genomic DNA contaminant.

14. The method of claim 1, wherein the composition has less than or about 0.1 EU/mg endotoxin, and less than or about 0.00002 % host cell RNA contaminant

15. The method of claim 1, wherein the composition has less than or about 0.1 EU/mg endotoxin, and less than or about 0.00005 % host cell protein contaminant.

16. The method of claim 1, wherein the composition has not more than 0.00002 % host cell RNA contaminant, and not more than 0.00005 % host cell protein contaminant.

17. The method of one of claims 1-16, which is amenable to scale-up for large-scale manufacture.

18. The method of any one of claims 1 to 17, further comprising a step of sterile filtration, formulation and filling of vials with the purified plasmid DNA.

19. The method according to any one of claims 1 to 17, wherein one or more of the chromatography steps is performed on a solid support comprising any organic, inorganic or composite material, porous, super-porous or non-porous support, suitable for chromatographic separations, which is derivatised with poly(alkene glycols), alkanes, alkenes, alkynes, arenes or other molecules that confer a hydrophobic character to the support.

20. The method according to any one of claims 1 to 17, wherein one or more of the chromatography steps is performed as displacement chromatography, simulated moving bed chromatography, continuous bed chromatography, fast protein liquid chromatography, or high performance liquid chromatography.

21. The method according of any of claims 1 to 17, wherein hydrophobic interaction chromatography is carried out in a fixed bed or an expanded bed.

**Patentansprüche**

1. Verfahren zur Herstellung einer Plasmid-DNA-Zusammensetzung pharmazeutischer Güteklasse, umfassend das Bereitstellen eines Zellextraktes, der Plasmid-DNA enthält, wobei die Zellen durch alkalische Lyse lysiert worden sind und die Zellmembranen und genomische DNA durch eine anfängliche Extraktion oder Filtration entfernt worden sind; und anschließendes Durchführen von mindestens drei Chromatographieschritten, umfassend in folgender Reihenfolge: Anionenaustauschchromatographie, Tripelhelix-Affinitätschromatographie, Hydrophobchromatographie, wobei die hergestellte Zusammensetzung weniger als etwa 0,0001 % Verunreinigung an genomischer Wirtszellen-DNA aufweist.

2. Verfahren von Anspruch 1, wobei dem ersten durchgeführten Chromatographieschritt eine Lysatfiltration vorangeht.

3. Verfahren von Anspruch 1, wobei dem ersten durchgeführten Chromatographieschritt eine Flockenentfernung vorangeht.

4. Verfahren von Anspruch 1, wobei die Zusammensetzung weniger als etwa 0,0001 % Wirtszell-RNA-Kontaminante enthält.

5. Verfahren von Anspruch 1, wobei die Zusammensetzung weniger als etwa 0,0001 % Wirtszell-Protein-Kontaminante enthält.

6. Verfahren von Anspruch 4, wobei die Zusammensetzung weniger als etwa 0,0001 % Wirtszell-Protein-Kontaminante enthält.

7. Verfahren von Anspruch 1, wobei die Zusammensetzung weniger als etwa 0,1 EU/mg Endotoxin aufweist.

8. Verfahren von Anspruch 6, wobei die Zusammensetzung weniger als etwa 0,1 EU/mg Endotoxin aufweist.

9. Verfahren von Anspruch 1, wobei die Zusammensetzung weniger als oder etwa 0,1 EU/mg Endotoxin, weniger als oder etwa 0,00008 % Wirtszell-Protein-Kontaminante, weniger als oder etwa 0,00008 % Wirtszell-RNA-Kontaminante und weniger als oder etwa 0,00008 % genomische Wirtszell-DNA-Kontaminante aufweist.

10. Verfahren von Anspruch 1, wobei die Zusammensetzung weniger als oder etwa 0,00005 % genomische Wirtszell-DNA-Kontaminante enthält.

11. Verfahren von Anspruch 1, wobei die Zusammensetzung weniger als oder etwa 0,00008 % genomische Wirtszell-DNA-Kontaminante enthält.

12. Verfahren von Anspruch 1, wobei die Zusammensetzung weniger als oder etwa 0,1 EU/mg Endotoxin und weniger als oder etwa 0,00005 % Wirtszell-Protein-Kontaminante aufweist.

13. Verfahren von Anspruch 12, wobei die Zusammensetzung weniger als oder etwa 0,00002 % Wirtszell-RNA-Kontaminate und weniger als oder etwa 0,00008 % genomische Wirtszell-DNA-Kontaminante aufweist.

14. Verfahren von Anspruch 1, wobei die Zusammensetzung weniger als oder etwa 0,1 EU/mg Endotoxin und weniger als oder etwa 0,00002 % Wirtszell-RNA-Kontaminante aufweist.

15. Verfahren von Anspruch 1, wobei die Zusammensetzung weniger als oder etwa 0,1 EU/mg Endotoxin und weniger als oder etwa 0,00005 % Wirtszell-Protein-Kontaminante aufweist.

16. Verfahren von Anspruch 1, wobei die Zusammensetzung nicht mehr als 0,00002 % Wirtszell-RNA-Kontaminante und nicht mehr als 0,00005 % Wirtszell-Protein-Kontaminante aufweist

17. Verfahren von einem der Ansprüche 1 - 16, welches einem Scale-up zur Herstellung in großem Maßstab zugänglich ist.

18. Verfahren von einem der Ansprüche 1 bis 17, welches ferner einen Schritt der sterilen Filtration, der Formulierung und des Befüllens von Vials mit der gereinigten Plasmid-DNA umfasst.

**19.** Verfahren gemäß einem der Ansprüche 1 bis 17, in dem einer oder mehrere der Chromatographieschritte auf einem festen Träger durchgeführt wird, welcher jedes beliebige organische, anorganische oder Compositmaterial, porösen, super-porösen oder nicht-porösen Träger, geeignet für chromatographische Trennungen, welcher mit Poly(alkenglycolen), Alkanen, Alkenen, Alkinen, Arenen oder anderen Molekülen, welche dem Träger einen hydrophoben Charakter geben, derivatisiert ist.

**20.** Verfahren gemäß einem der Ansprüche 1 bis 17, in dem einer oder mehrere der Chromatographieschritte als Verdrängungschromatographie, simulierte Bewegt-Bett-Chromatographie, Chromatographie mit kontinuierlichem Bett, FPLC (Fast Protein Liquid-Chromatographie) oder Hochleistungschromatographie durchgeführt wird.

**21.** Verfahren gemäß einem der Ansprüche 1 bis 17, in dem die Hydrophobchromatographie in einem Festbett oder einem expandierten Bett durchgeführt wird.

## Revendications

**1.** Procédé de préparation d'une composition d'ADN plasmidique de qualité pharmaceutique comprenant la mise à disposition d'un extrait de cellules contenant de l'ADN plasmidique, **caractérisé en ce que** les cellules ont été lysées par lyse alcaline et les membranes cellulaires et l'ADN génomique ont été éliminés par une extraction ou filtration initiale ; et ensuite la réalisation d'au moins trois étapes de chromatographie comprenant dans l'ordre suivant : la chromatographie d'échange d'anions, la chromatographie d'affinité à triple hélice, et la chromatographie d'interaction hydrophobe, où la composition préparée a moins d'environ 0,0001 % de contamination par l'ADN génomique de cellule hôte.

**2.** Procédé de la revendication 1, **caractérisé en ce que** la première étape de chromatographie effectuée est précédée par une filtration du lysat.

**3.** Procédé de la revendication 1, **caractérisé en ce que** la première étape de chromatographie effectuée est précédée par une élimination des floculats.

**4.** Procédé de la revendication 1, **caractérisé en ce que** la composition a moins d'environ 0,0001 % de contaminant d'ARN de cellule hôte.

**5.** Procédé de la revendication 1, **caractérisé en ce que** la composition a moins d'environ 0,0001 % de contaminant de protéine de cellule hôte.

**6.** Procédé de la revendication 4, **caractérisé en ce que** la composition a moins d'environ 0,0001 % de contamination de protéine de cellule hôte.

**7.** Procédé de la revendication 1, **caractérisé en ce que** la composition a moins d'environ 0,1 UE/mg d'endotoxine.

**8.** Procédé de la revendication 6, **caractérisé en ce que** la composition a moins d'environ 0,1 UE/mg d'endotoxine.

**9.** Procédé de la revendication 1, **caractérisé en ce que** la composition a moins de ou environ 0,1 UE/mg d'endotoxine, moins de ou environ 0,00008 % de contaminant de protéine de cellule hôte, moins de ou environ 0,00008 % de contaminant d'ARN de cellule hôte, et moins de ou environ 0,00008 % de contaminant d'ADN génomique de cellule hôte.

**10.** Procédé de la revendication 1, **caractérisé en ce que** la composition a moins de ou environ 0,00005 % de contaminant d'ADN génomique de cellule hôte.

**11.** Procédé de la revendication 1, **caractérisé en ce que** la composition a moins de ou environ 0,00008 % de contaminant d'ADN génomique de cellule hôte.

**12.** Procédé de la revendication 1, **caractérisé en ce que** la composition a moins de ou environ 0,1 UE/mg d'endotoxine et moins de ou environ 0,00005 % de contaminant de protéine de cellule hôte.

**13.** Procédé de la revendication 12, **caractérisé en ce que** la composition a moins de ou environ 0,00002 % de

contaminant d'ARN de cellule hôte, et moins de ou environ 0,00008 % de contaminant d'ADN génomique de cellule hôte.

**14.** Procédé de la revendication 1, **caractérisé en ce que** la composition a moins de ou environ 0,1 UE/mg d'endotoxine, et moins de ou environ 0,00002 % de contaminant d'ARN de cellule hôte.

**15.** Procédé de la revendication 1, **caractérisé en ce que** la composition a moins de ou environ 0,1 UE/mg d'endotoxine, et moins de ou environ 0,00005 % de contaminant de protéine de cellule hôte.

**16.** Procédé de la revendication 1, **caractérisé en ce que** la composition a moins de ou environ 0,00002 % de contaminant d'ARN de cellule hôte, et pas plus de 0,00005 % de contaminant de protéine de cellule hôte.

**17.** Procédé de l'une des revendications 1 à 16, qui est adapté pour être mis à l'échelle pour la fabrication à grande échelle.

**18.** Procédé de l'une quelconque des revendications 1 à 17, comprenant en outre une étape de filtration stérile, formulation et remplissage de flacons avec l'ADN plasmidique purifié.

**19.** Procédé selon l'une quelconque des revendications 1 à 17, **caractérisé en ce qu'**une ou plusieurs des étapes de chromatographie est réalisée sur un support solide comprenant un support poreux, super-poreux ou non poreux de matériau organique, inorganique ou composite quelconque, adapté pour les séparations chromatographiques, qui est dérivé des poly(alcène)glycols, des alcanes, des alcènes, des alcynes, des arènes ou d'autres molécules qui confèrent un caractère hydrophobe au support.

**20.** Procédé selon l'une quelconque des revendications 1 à 17, **caractérisé en ce qu'**une ou plusieurs des étapes de chromatographie est réalisée en tant que chromatographie de déplacement, chromatographie sur lit mobile simulé, chromatographie sur lit continu, chromatographie liquide de protéine rapide, ou chromatographie liquide haute performance.

**21.** Procédé selon l'une quelconque des revendications 1 à 17, **caractérisé en ce que** la chromatographie d'interaction hydrophobe est réalisée dans un lit fixe ou un lit expansé.

FIGURE 1

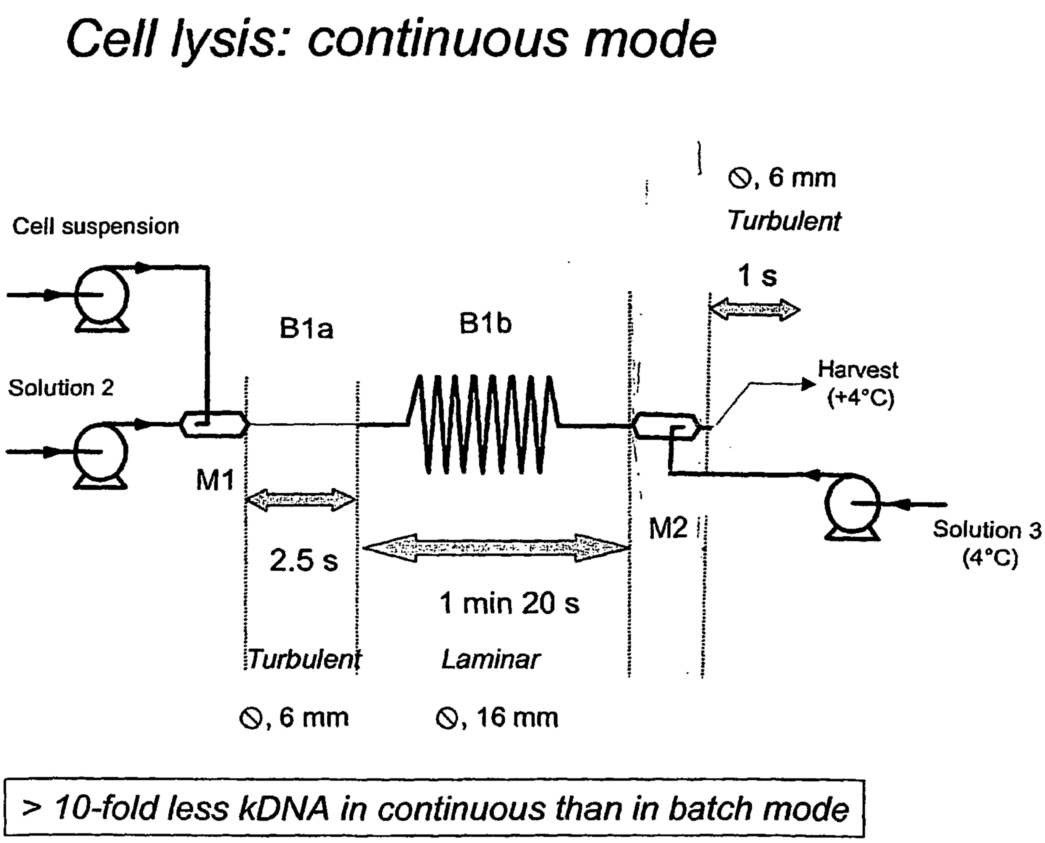
Cell lysis: continuous mode
Cell suspension
Solution 2
M1
B1a
B1b
2.5 s
1 min 20 s
Turbulent
Laminar
⦸, 6 mm
⦸, 16 mm
M2
⦸, 6 mm
Turbulent
1 s
Harvest
(+4°C)
Solution 3
(4°C)
> 10-fold less kDNA in continuous than in batch mode

FIGURE 2

General view of mixer M1

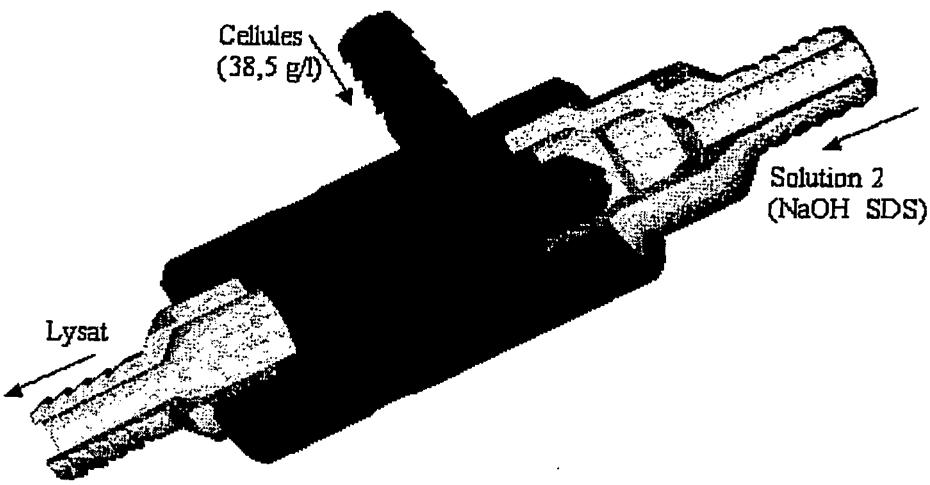

## FIGURE 3

| Sequence | Content of genomic DNA (%) | RNA (%) | Protein (%) | Endotox. (EU/mg of plasmid) | ccc form (%) | Comments |
|---|---|---|---|---|---|---|
| Lysis | 500 | > 1000 | > 2000 | > 100 000 | ≈ 95 | |
| Anion exchange chromatography (AEC) [FractogelTMAE (HiCap)] | 10 to 50 | 5 to 20 | 0.0002 | 20 to > 200 | ≈ 95 | Very efficient protein removal (> $10^6$-fold)<br>Efficient endotoxin removal (1000-fold) |
| Anion exchange chromatography and Triple helix affinity chromatography (THAC-Sephacryl S1000) | 0.3 to 1.0 | 0.002 to 0.01 | < 0.0001 | 10 to 200 | ≈ 95 | Very efficient RNA removal (1000-fold)<br>Efficient genomic DNA removal (100-fold)<br>Poor endotoxin removal (< 10-fold)<br>No open circular plasmid form removal |
| Anion exchange chromatography and hydrophobic interaction chromatography (HIC) [Toyopearl Butyl (650s)] | 0.2 to 0.6 | < 0.01 | ND | < 0.2 | > 99 | Very efficient oc plasmid form removal (10-fold)<br>Efficient genomic DNA and RNA removal (100-fold)<br>Efficient endotoxin removal (1000-fold) |
| Anion exchange chromatography , and Triple helix affinity chromatography and hydrophobic interaction chromatography in combination (AEC / THAC / HIC | < 0.00008 | < 0.00002 | < 0.00005 | < 0.1 | > 99 | Very efficient removal of genomic DNA, RNA, protein, endotoxin, and oc plasmid form |

FIGURE 4

| Methods of separation and purification of plasmid DNA | Genomic DNA (%) | RNA (%) | Protein (%) | Endotoxin (EU/mg) |
|---|---|---|---|---|
| $CaCl_2$ / AEC / AEC | < 1 | < 0.1 | < 2.2 | < 1.5 |
| HIC / HIC | < 1 | < 1 | < 1 | < 5 |
| AEC/HAC | | | < 0.1 | < 0.1 |
| AEC / SEC | < 1 | < 0.2 | < 1 | < 100 |
| UF / AEC | < 1 | < 1 | < 1 | < 1 |
| RPC / AEC | < 0.2 to 1.0 | ND | < 0.1 to 1 | < 5 |
| UF / AEC | < 1 | < 2 | < 0.1 | < 100 |
| Heat lysis / AEC / RPC | 3 | < 1 | < 1 | 2.8 |
| Filtration/RNase/AEC | < 2 | < 0.1 | < 0.1 | < 20 |
| Detergent / precipitation / SEC | < 1 | < 1 | < 0.5 | < 5 |
| PEG / AEC / SEC<br>Filtration (diatomaceous earth) / SEC / precipitation | < 1 | ND | ND | < 100 |
| THAC<br>HAC / IEC or HAC / THAC | < 0.01 | | | < 10 |
| AEC / THAC / HIC | < 0.00008 | < 0.00002 | < 0.00005 | < 0.1 |

FIGURE 5

A) Formation of open circular and depurinated forms at +25°C (experiments with LS06).

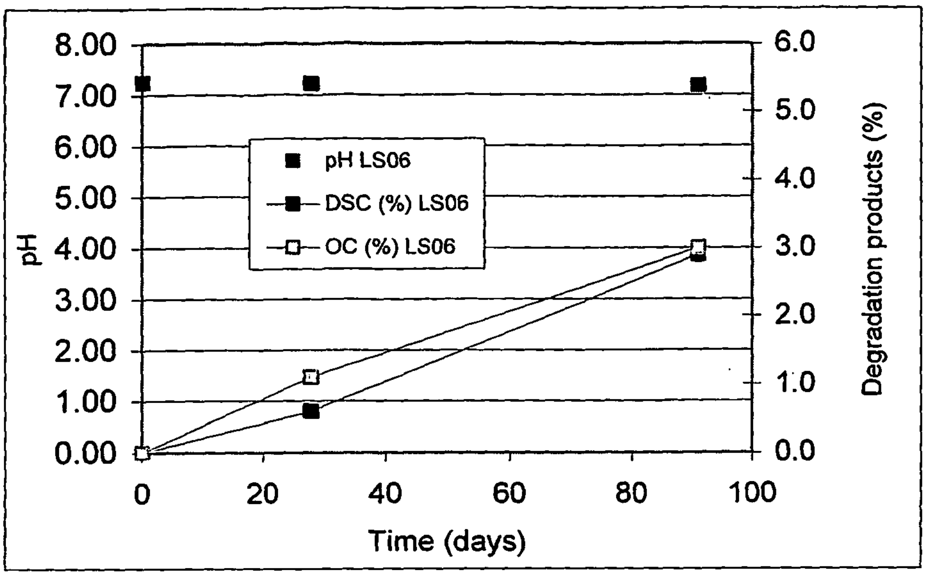


B) Formation of open circular and depurinated forms at +5°C (experiments with LS06).

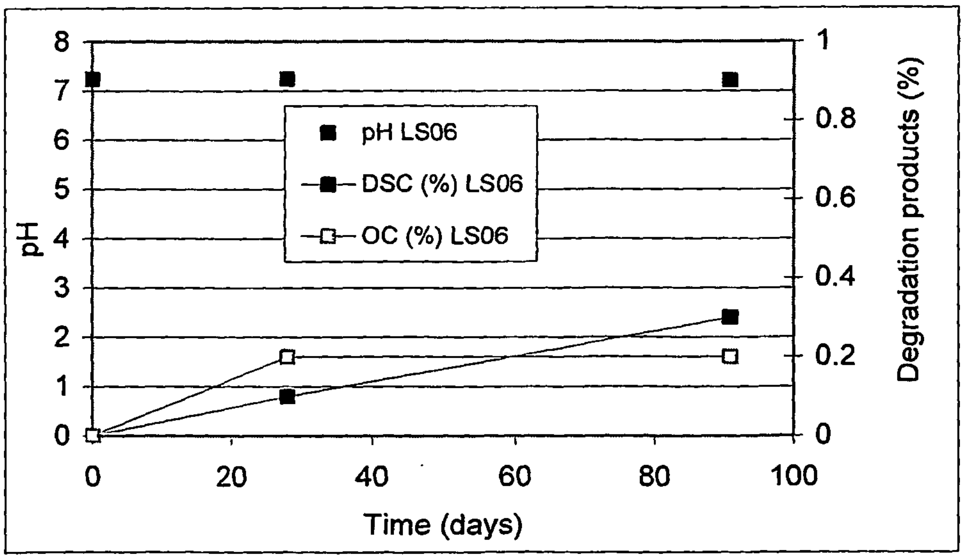

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6319672 B
- US 6287762 B
- WO 0277274 A
- US 2004142452 A
- US 2003161844 A
- US 6441160 B
- WO 9602658 A
- WO 9723601 A
- US 20031618445 A
- WO 04033664 A
- WO 9800815 A
- WO 9929832 A, D.S. McNeilly
- US 6214568 B, D.S. McNeilly
- EP 2004011437 W
- US 60563008 B


### Non-patent literature cited in the description


- **Duarte et al.** *Journal of Chromatography A,* 1998, vol. 606, 31-45
- **Prazeres, D.M.** *Biotechnology Techniques,* June 1997, vol. 1 (6), 417-420
- **ferrreira et al.** *Trends in biotechnology,* 2000, 380-388
- **Stadles et al.** *J. of Gene Medecine,* 2004, S54-S66
- **Maniatis et al.** Molecular Cloning, A Laboratory Manual. Cold Spring Harbor Laboratory Press, 1989
- **Birnboim ; Doly.** *Nucleic Acids Research,* 1979, vol. 7, 1513
- **Curless et al.** *Bioeng.,* 1991, vol. 38, 1082-1090
- Fermentation Technology Using Recombinant Microorganisms. **Fieschio et al.** In Biotechnology. VCH Verlagsgesellschaft mbH, 1989, vol. 7b, 117-140
- **Rajagopal et al.** *Biochem,* 1989, vol. 28, 7859
- **Vasquez et al.** *Biochemistry,* 1995, vol. 34, 7243-7251
- **Beal ; Dervan.** *Science,* 1991, vol. 251, 1360-1363
- **Beal ; Dervan.** *J. Am. Chem. Soc.,* 1992, vol. 114, 4976-4982
- **Jayasena ; Johnston.** *Nucleic Acids Res.,* 1992, vol. 20, 5279-5288
- **Duval-Valentin et al.** *Proc. Natl. Acad. Sci. USA,* 1992, vol. 89, 504-508
- **Lacatena et al.** *Nature,* 1981, vol. 294, 623
- **Levchenko et al.** *Nucleic Acids Res.,* 1996, vol. 24, 1936
- **Kiessling et al.** *Biochemistry,* 1992, vol. 31, 2829-2834
- **Xodo et al.** *Nucleic Acids Res.,* 1994, vol. 22, 3322-3330
- **Matteucci et al.** *J. Am. Chem. Soc.,* 1991, vol. 113, 7767-7768
- **Le Doan et al.** *Nucleic Acids Res.,* 1987, vol. 15, 7749-7760
- *J. Am. Chem. Soc.,* 1994, vol. 116, 3143-3144
- **Sun ; Hélène.** *Curr. Opinion Struct. Biol.,* vol. 116, 3143-3144
- **Nielsen et al.** *Science,* 1991, vol. 254, 1497-1500
- **Kim et al.** *J. Am. Chem. Soc.,* 1993, vol. 115, 6477-6481
- *Proc. Natl. Acad. Sci. USA,* 1995, vol. 92, 6097-6101
- **Povsic ; Dervan.** *J. Am. Chem. Soc.,* 1989, vol. 111, 3059-3061
- **Milligan et al.** *Nucleic Acids Res.,* 1993, vol. 21, 327-333
- **Koh ; Dervan.** *J. Am. Chem. Soc.,* 1992, vol. 114, 1470-1478
- **Sun ; Hélène.** *Curr. Opinion Struct. Biol.,* 1993, vol. 3, 345-356
- **Toshio Nambara ; Nobuo Ikegawa.** Latest High-Speed Liquid Chromatography. Tokyo Hirokawa Bookstore, 1988, 289 ff
- **Camerota et al.** *J Vasc. Surg.,* 2002, vol. 35 (5), 930-936
- **Ausubel F.M. ; R. Brent ; R.E. Kinston ; D.D. Moore ; J.A. Smith ; J.G. Seidman ; K. Struhl.** Current protocols in molecular biology. John Willey and Sons, 1987
- **Sinha, N.D. ; J. Biernat ; J. McManus ; H. Köster.** Polymer support oligonucleotide synthesis, XVIII: Use of β-cyanoethyl-N,N-dialkylamino-/N-morpholino phosphoramidite of deoxynucleosides for the synthesis of DNA fragments simplifying deprotection and isolation of the final product. *Nucl. Acids Res.,* 1984, vol. 12, 4539-4557

- **Giles, J.W.** Advances in automated DNA synthesis. *Am. Biotechnol.,* November 1985
- **Viera J. ; J. Messing.** The pUC plasmids, an M13mp7-derived system for insertion mutagenesis and sequencing with synthetic universal primers. *Gene,* 1982, vol. 19, 259-268
- **Debouck et al.** *Nucleic Acids Res.,* 1985, vol. 13, 1841-1853
- *Gene,* vol. 19, 259-268
- **Hornsey et al.** *J. Immunol. Methods,* 1986, vol. 93, 83-88
- **Soubrier et al.** *Gene Therapy,* 1999, vol. 6, 1482-1488
- **Marquet et al.** *BioPharm,* 1995, vol. 8, 26-37
- **H.C. Birnboim ; J. Doly.** A rapid alkaline extraction procedure for screening recombinant plasmid DNA. *Nucleic Acid Research,* 1979, vol. 7 (6), 1513-1523
- **D. Stephenson ; F. Norman ; R.H. Cumming.** Shear thickening of DNA in SDS lysates. *Bioseparation,* 1993, vol. 3, 285-289
- **M.S. Levy ; L.A.S. Ciccolini ; S.S.S. Yim ; J.T. Tsai ; N. Titchener-Hooker ; P. Ayazi Shamlou ; P. Dunnill.** The effects of material properties and fluid flow intensity on plasmid DNA recovery during cell lysis. *Chemical Engineering Science,* 1999, vol. 54, 3171-3178